(19) Europäisches Patentamt
European Patent Office
Office européen des brevets

(11) Publication number: **0 048 136**
**B1**

(12) **EUROPEAN PATENT SPECIFICATION**

(45) Date of publication of patent specification: **11.06.86**

(51) Int. Cl.$^4$: **C 07 C 67/333,** C 07 C 143/68

(21) Application number: **81304154.8**

(22) Date of filing: **10.09.81**

(54) **Process for preparing alpha-aromatic group substituted alkanoic acids or esters thereof.**

(30) Priority: **11.09.80 JP 125355/80
15.10.80 JP 143042/80
10.11.80 JP 157049/80
30.01.81 JP 11700/81
15.06.81 JP 90979/81**

(43) Date of publication of application:
**24.03.82 Bulletin 82/12**

(45) Publication of the grant of the patent:
**11.06.86 Bulletin 86/24**

(84) Designated Contracting States:
**AT BE CH DE FR GB IT LI LU NL SE**

(56) References cited:
**EP-A-0 034 871
EP-A-0 035 305
GB-A-2 042 543**

**BERICHTE DER DEUTSCHEN CHEMISCHEN GESELLSCHAFT 1927, pages 656-665, ZOLTAN FÖLDI: "Über die thermische Zersetzung der Sulfonsäure-ester"**

(73) Proprietor: **SYNTEX PHARMACEUTICALS INTERNATIONAL LIMITED
Corner House Church Street
Hamilton 5 (BM)**

(72) Inventor: **Tsuchihashi, Genichi
2-14-7, Sakuragaoka
Tama-shi Tokyo (JP)**
Inventor: **Mitamura, Shuichi
1-9-2, Minamidai
Sagamihara-shi Kanagawa-ken (JP)**
Inventor: **Kitajima, Kouji
1-9-1-203, Minamidai
Sagamihara-shi Kanagawa-ken (JP)**

(74) Representative: **Myerscough, Philip Boyd et al
J.A.Kemp & Co. 14, South Square Gray's Inn
London, WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 048 136 B1

**Description**

This invention relates to a process for preparing an alpha-aromatic group substituted alkanoic acid or ester thereof of the general formula

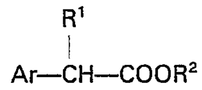

(I)

wherein Ar represents an aromatic group, $R^1$ represents a hydrogen atom or a saturated aliphatic group, or Ar and $R^1$ may form a condensed ring together with the carbon atom to which they are bonded, and $R^2$ represents a hydrogen atom, an alkyl group, or a hydroxyalkyl group.

Many of the compounds of general formula (I) are commercially valuable. For example, alpha-(4-isobutylphenyl) propionic acid corresponding to a compound of general formula (I) in which Ar is a 4-isobutylphenol group, $R^1$ is a methyl group, and $R^2$ is a hydrogen atom, is known as "Ibuprofen" which is an anti-inflammatory drug. Alpha - (6 - methoxy - 2 - naphthyl)propionic acid corresponding to a compound of general formula (I) in which Ar is a 6 - methoxy - 2 - naphthyl group, $R^1$ is a methyl group, and $R^2$ is a hydrogen atom is known as "Naproxen". Alpha - (4 - difluoromethoxyphenyl)isovaleric acid, corresponding to a compound of general formula (I) in which, Ar is a 4-difluoromethoxyphenyl group, $R^1$ is an isopropyl group, and $R^2$ is a hydrogen atom is very effective as an acid moiety of pyrethroid insecticides.

Many processes have been known heretofore for the production of alpha-aromatic group substituted alkanoic acids. They may be typically described below with reference to the production of alpha - (4 - isobutylphenyl)propionic acid (Ibuprofen).

(1) A process which comprises reacting a 4-isobutylphenylacetic ester, produced in two steps from 4-isobutylacetophenone, with an alkyl carbonate in the presence of a base to form the corresponding malonic ester, methylating the malonic ester with methyl iodide, hydrolyzing the methylation product, and subsequently thermally decomposing the product to obtain the desired propionic acid (GB—A—971,700).

(2) A process which comprises converting 4-isobutylacetophenone into the corresponding hydantoin by the action of potassium cyanide and ammonium carbonate, hydrolyzing the hydantoin to an alpha-amino acid, alkylating it to a dialkylamino compound, and reducing it to form alpha - (4 - isobutylphenyl)propionic acid (GB—A—1167192).

(3) A process which comprises subjecting 4-isobutylacetophenone and a monochloroacetic ester to the Darzens reaction to form the corresponding epoxycarboxylic ester, hydrolyzing the ester, decarboxylating the hydrolyzed product to form alpha - (4 - isobutylphenyl)propionaldehyde, and the oxidizing the aldehyde to the desired propionic acid (GB—A—1160725).

(4) A process which comprises condensing 4-isobutylbenzaldehyde with formaldehyde mercaptal S-oxide to form a ketene mercaptal S-oxide, reacting it with thionyl chloride to form an alpha-chloroketene mercaptal, alcoholizing it to form an alpha - (4 - isobutylphenyl)alpha-alkylthioacetic ester, and subjecting the ester to methylation, hydrolysis, and reductive desulfurization in this sequence to obtain the desired propionic acid (US—A—4,242,519).

The processes (1) and (4) include many process steps and are not industrially advantageous. The process (2) is industrially disadvantageous because it employs a poisonous substance such as potassium cyanide.

The processes (1) and (3) are economically disadvantageous because the ethoxycarbonyl group introduced in the initial step is removed by performing decarboxylation in the final step.

It is apparent therefore that the provision of a novel and industrially advantageous process for producing the compounds of general formula (I) will greatly contribute to the development of technology in the art.

According to the present invention, there is provided a process for preparing an alpha-aromatic group substituted alkanoic acid or its ester of the general formula

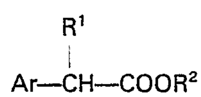

(I)

wherein Ar represents an aromatic group, $R^1$ represents a hydrogen atom or a saturated aliphatic group, or Ar and $R^1$ may form a condensed ring together with the carbon atom to which they are bonded, and $R^2$ represents a hydrogen atom, an alkyl group, or a hydroxyalkyl group, characterized in that an alpha-sulfonyloxyketone acetal of the general formula

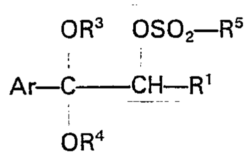

(II)

wherein $R^3$ and $R^4$, independently from each other, represent an alkyl group, or together represent an alkylene group, $R^5$ represents a substituted or unsubstituted alkyl group or an aromatic group, and Ar and $R^1$ are as defined above, is hydrolyzed, or treated with an agent having affinity for oxygen.

The term "aromatic group", as used in the present specification and appended claims, is to be taken in its broadest meaning, and denotes a group of a cyclic compound having aromaticity. The aromaticity means a phenomenon wherein the ring is stabilized by the delocalization of π electrons. Generally, a ring having (4n+2) conjugated π electrons is stable and exhibits aromaticity. Thus, the aromatic group, as used herein, refers to a group of compounds having (4n+2) conjugated π electrons in the main ring. It can be classified roughly into aryl groups optionally having at least one substituent and heretoaromatic groups optionally having at least one substituent, which are described in detail below.

(a) Aryl groups optionally having at least one substituent

The aryl groups are aromatic hydrocarbon groups of the monocyclic, polycyclic or condensed polycyclic type, and include, for example, phenyl, biphenyl and naphthyl.

The aryl groups may be unsubstituted, or have one or more substituents on the aromatic ring. Specific examples of the substituents include halogen atoms such as chlorine, bromine, fluorine, and iodine; lower alkyl groups such as methyl, ethyl, n-propyl, isopropyl, n-butyl, isobutyl, and tert-butyl; cycloalkyl groups such as cyclohexyl and cyclopentyl; aralkyl groups such as benzyl; lower alkoxy groups such as methoxy, ethoxy, n-propoxy and isopropoxy; lower alkylthio groups such as methylthio, ethylthio, n-propylthio, isopropylthio, and butylthio; arylthio groups such as phenylthio, tolylthio, and naphthylthio; alkenylthio groups such as alkylthio; aralkylthio such as benzylthio; acyloxy groups such as acetoxy; aroyloxy groups such as benzoyloxy; silyloxy groups such as trimethylsilyloxy; lower alkenyl groups such as allyl and prenyl [$(CH_3)_2C{=}CH{-}CH_2{-}$]; lower alkenyloxy groups such as allyloxy; aralkyloxy groups such as benzyloxy and phenethyloxy; aryloxy groups such as phenoxy; lower haloalkyl groups such as trifluoroethyl and trifluoropropyl; lower haloalkoxy groups such as difluoromethoxy and trifluoromethoxy; 4- to 6-membered heterocyclic groups such as indolinyl, oxo-isoindolynyl thienyl, piperidino and phthalimido; 4- to 6-membered heterocycloxy groups such as thiazoyloxy, and pyridyloxy; a nitro group; aroyl groups such as benzoyl; acylamino groups such as acetylamino and propionylamino; aroylamino groups such as benzoylamino; and dialkylamino groups such as dimethylamino and dibenzylamino. When these substituents are present, 1 to 5, preferably 1 to 3, of them are preferably present on the aromatic ring.

Specific examples of aryl groups having such substituents on the aromatic ring include chlorophenyl, fluorophenyl, bromophenyl, iodophenyl, tolyl, ethylphenyl, isopropylphenyl, isobutylphenyl, tert-butylphenyl, cyclohexylphenyl, methoxyphenyl, ethoxyphenyl, isopropoxyphenyl, methylthiophenyl, ethylthiophenyl, n-propylthiophenyl, isopropylthiophenyl, butylthiophenyl, phenylthiophenyl, tolylthiophenyl, allylthiophenyl, benzylthiophenyl, acetoxyphenyl, trimethylsilyloxyphenyl, benzoyloxyphenyl, benzylphenyl, prenylphenyl, allyloxyphenyl, benzyloxyphenyl phenoxyphenyl, tetrafluoroethoxyphenyl, trifluoroethylphenyl, trifluoromethoxyphenyl, difluoromethoxyphenyl, oxo-isoindolinylphenyl, thioazolyloxyphenyl, nitrophenyl, benzoylphenyl, acetylaminophenyl, piperidinophenyl, fluorobiphenyl, acetylaminobiphenyl, and methoxynaphthyl, methylthienylphenyl, acetylamino - chloro - phenyl - chloro - cyclohexyl - phenyl.

(b) Heteroaromatic groups optionally having at least one substituent

The heteroaromatic group may be of any of the monocyclic or condensed polycyclic type. The heteroatom of the heteroaromatic ring may be nitrogen, oxygen or sulfur. The heteroaromatic ring contains generally 1 to 4, preferably 1 to 3, such hetero atoms, and may be generally 5- or 14-membered, preferably 5- to 9-membered. Specific examples of such heteroaromatic groups are thienyl, furyl, pyrrolyl, indolyl, phenothiazinyl, pyridyl, thiazolyl, and benzothiazolyl.

The heteroaromatic group may be unsubstituted, or may contain one or more substituents on the ring. Examples of substituents which may be present on the heterocyclic aromatic ring are lower alkyl groups such as methyl, ethyl, propyl and butyl; aryl groups such as phenyl and fluorophenyl; halogen atoms such as chlorine and fluorine; lower alkoxy groups such as methoxy, ethoxy and propoxy; aryloxyl groups such as a phenoxy lower alkyl groups such as methyl, ethyl, propyl, and butyl; aralkyl groups such as benzyl; aryl groups such as phenyl, tolyl, and fluorophenyl; halogen atoms such as fluorine, chlorine and bromine; lower alkoxy groups such as methoxy, ethoxy, and propoxy; cycloalkyl groups such as cyclohexyl and cyclopentyl; lower alkenyl groups such as allyl and prenyl; lower alkenyloxy groups such as allyloxy; aralkyloxy groups such as benzyloxy; aryloxy groups such as phenoxy; lower haloalkyl groups such as trifluoromethyl; lower alkylthio groups such as methylthio; arylthio groups such as phenylthio; aroyl groups such as benzoyl, toluoyl, and chlorobenzoyl; acyl groups such as acetyl; lower haloalkyl groups such as trifluoromethyl; and cycloalkyl groups such as cyclohexyl.

When these substituents are present, 1 to 8, preferably 1 to 3, of them are desirably present.

Thus, examples of heteroaromatic groups having such substituents include methylphenothiazinyl, methoxy-methyl-phenothiazinyl, methylpyrrolyl, toluoyl-methyl-pyrrolyl, phenylthienyl, bromothienyl, trifluoromethylthienyl, benzoylthienyl, cyclohexylthienyl, phenoxythienyl, methyl-methoxy-indolyl, chlorobenzoyl-indolyl, acetylpyrrolyl, methyl-toluoyl-pyrrolyl, benzylpyrrolyl.

The term "saturated aliphatic group, as used in the present specification and the appended claims,

denotes a linear, branched, or cyclic saturated aliphatic hydrocarbon group which may generally contain 1 to 10 carbon atoms, preferably 1 to 6 carbon atoms. Examples of such saturated aliphatic groups include linear or branched alkyl groups having 1 to 6 carbon atoms, especially lower alkyl groups, such as methyl, ethyl, n-propyl, isopropyl, n-butyl, isobutyl, tert-butyl, n-pentyl, isoamyl, and n-hexyl, and cycloalkyl groups having 3 to 10 carbon atoms, especially cycloalkyl groups having 3 to 7 carbon atoms, such as cyclopropyl, cyclopentyl, cyclohexyl, cycloheptyl.

Examples of the substituents which may be present on the alkyl group in the "substituted alkyl group" are halogen atoms such as chlorine, bromine and fluorine; aryl groups such as phenyl; alkoxy groups such as methoxy and ethoxy; and alicyclic groups such as [1R, 4R] or [1S, 4S] - 7,7 - dimethyl - 2 - oxobicyclo[2,2,1]heptan - 1 - yl. Specific examples of such substituted alkyl groups include trifluoromethyl, d- or l-10-camphoryl, and benzyl.

The "alkyl group" and "alkylene group", used in the present specification and appended claims, may be any one of linear or branched types, and the alkylene groups are preferably lower alkylene groups such as ethylene propylene, butylene, trimethylene, or tetramethylene.

The term "lower", as used in the present specification and appended claims to qualify a group or a compound means that the group or compound so qualified has not more than 6, preferably not more than 4, carbon atoms.

According to one embodiment of the process of this invention, the alpha-sulfonyloxyketone acetal of general formula (II) is hydrolyzed.

It has been found that when the compound of general formula (II) which is a specified acetal compound having a sulfonyloxy group ($OSO_2R^5$) at the 2-position and an aromatic group (Ar) at the 1-position is hydrolyzed, a unique reaction takes place in which the sulfonyloxy group is split off and the aromatic group is shifted to the 2-position to give an alpha-aromatic group substituted alkanoic acid of general formula (I).

The hydrolysis reaction may be carried out in the absence of a solvent. Desirably, however, it is carried out generally in an inert solvent. Examples of the inert solvent are aprotic polar solvents such as dimethyl formamide (DMF), dimethyl sulfoxide (DMSO), 1,3 - dimethyl - 2 - imidazolidinone (DMI), 1,4-dioxane, tetrahydrofuran (THF) diethylene glycol dimethyl ether (diglyme), hexamethylphosphoric triamide (HMPA), 1,2-dimethoxyethane, and pyridino; and protic polar solvents such as methanol, ethanol, ethylene glycol, and acetic acid. These solvents may be used solely or as a mixture of two or more.

The reaction temperature is not critical, and can be varied widely according to the type of the starting material (II), etc. Generally, temperatures between about 0°C and about 250°C, preferably room temperature to about 200°C, can be used. In order to promote the reaction, the reaction is conveniently carried out at an elevated temperature, preferably from about 40°C to the refluxing temperature of the reaction mixture, more preferably from about 50°C to about 180°C. The reaction may be carried out at atmospheric or elevated pressures.

Water required for hydrolysis of the compound of formula (II) may be incorporated in advance in a solvent of the type exemplified hereinabove and the compound of formula (II) may be mixed in the solvent to perform the reaction. Or it is possible to mix the compound of formula (II) in the solvent, add water to the mixture, and react the water with the compound (II).

Alternatively, the compound of formula (II) is heated to a temperature within the above range in the above solvent in anhydrous conditions, and then water is added to hydrolyze the compound of formula (II). (See Examples 31 and 35 below).

From the standpoint of the ease of operation, it is convenient to introduce the compound of formula (II) into a mixture of water and the above polar solvent.

The amount of water required for hydrolysis is not critical, and can be varied widely depending upon the type of the compound (II) used, the reaction conditions, etc. Generally, it is used in an amount of at least 1 mole, preferably at least 5 moles, per mole of the compound of formula (II). If it is used in too large an amount, the solubility of the compound of formula (II) decreases. Hence, it is preferable not to use water in too excessive an amount.

In order to prevent the undesired cleavage of the acetal moiety of the compound of formula (II) by an acid, the hydrolysis is preferably carried out generally under neutral or basic (pH about 7—14) conditions.

Since the sulfonyloxy group ($—OSO_2—R^5$) at the 2-position is split off as sulfonic acid $R^5SO_3H$ in the hydrolysis reaction, it is convenient to cause a base to be present in the reaction system in order to maintain the reaction system under neutral to basic conditions during the proceeding of the reaction. Examples of the base which can be used for this purpose include alkali metal hydroxides such as potassium hydroxide and sodium hydroxide; alkaline earth metal hydroxides such as magnesium hydroxide and calcium hydroxide; alkali metal carbonates such as potassium carbonate and sodium carbonate; alkaline earth metal carbonates such as magnesium and calcium carbonate; alkali metal bicarbonates such as potassium and sodium bicarbonate; alkali metal carboxylates such as sodium formate, sodium acetate, potassium acetate and sodium propionate; alkalimetal phosphates such as sodium and potassium phosphate; and organic tertiary amines such as pyridine, triethylamine, and tributylamine. These inorganic or organic bases are conveniently used in an amount of generally at least 1 equivalent, preferably 1 to 10 equivalents, per mole of the compound of formula (II).

The hydrolysis reaction can be terminated generally within about 1 to about 250 hours although this depends upon the type of the starting compound of formula (II) and the reaction conditions.

In the above hydrolysis reaction, the sulfonyloxy group ($—OSO_2—R^5$) at the 2-position of the compound of formula (II) is split off, and the aromatic group (Ar) at the 1-position is shifted to the 2-position. At the same time, either one of the acetal groups $—OR^3$ and $—OR^4$ is split off, and the remaining one acetal group forms the group $—OR^2$ in the compound of formula (I) (in which $R^2$ is an alkyl group). Depending upon the conditions used in the hydrolysis, especially under strongly basic conditions, the ester (I) formed [the compound of formula (I) in which $R^2$ is an alkyl group] further undergoes hydrolysis to form the compound of formula (I) in which $R^2$ is a hydrogen atom.

When the compound of formula (II) in which $R^3$ and $R^4$ together form an alkylene group hydrolyzed, a corresponding compound of formula (I) is obtained in which $R^2$ is a hydroxyalkyl group.

According to another embodiment of the process of the invention, the compound of general formula (II) is treated with an agent having affinity for oxygen. It has been found that as a result of this treatment, the sulfonyloxy group ($—OSO_2—R^5$) at the 2-position of the compound of general formula (II) is split off and the aromatic group (Ar) at the 1-position is shifted to the 2-position, to give the compound of formula (I). In this reaction, too, either one of the group $—OR^3$ or $—OR^4$ of the compound of general formula (II) forms the $—OR^2$ group of the product of formula (I). Hence, the product of this reaction is the ester of formula (I) in which $R^2$ is an alkyl group.

The term "agent having affinity for oxygen", as used herein, denotes a compound having the ability to coordinate in such a way as to accept a lone electron pair of oxygen atom. Specific examples of the agent having affinity for oxygen include the following.

(a) Iodotrialkylsilanes of the formula

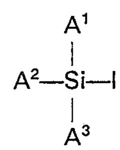

(III)

wherein $A^1$, $A^2$ and $A^3$ are identical or different and each represent an alkyl group, especially a lower alkyl group, such as iodotrimethylsilane and iodotriethylsilane.

(b) Trialkylsilyl perfluoroalkylsulfonates of the formula

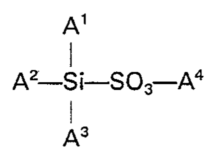

(IV)

wherein $A^4$ represents a perfluoroalkyl group, particularly a lower perfluoroalkyl group, and $A^1$, $A^2$, and $A^3$ are as defined hereinabove, such as trimethylsilyl trifluoromethanesulfonate, and trimethylsilyl pentafluoroethanesulfone.

(c) Lewis acids such as aluminum chloride, aluminum bromide, zinc chloride, tin chloride, titanium chloride, boron fluoride, and iron chloride.

These agents having affinity for oxygen may be used solely or as a mixture of two or more. Especially preferred agents having affinity for oxygen used in this invention are iodotrimethylsilane, trimethylsilyl trifluoromethanesulfonate, iron chloride, aluminum chloride, and tin chloride.

The amount of the agent having affinity for oxygen used is not strictly limited, and can be varied widely according to the type of the compound of formula (II), and/or the type of the agent. Generally, it is used in an amount of at least 0.1 mole, preferably 0.2 to 5.0 moles, more preferably 1.0 to 2.0 moles, per mole of the compound of formula (II).

The treatment of the compound of formula (II) with the agent having affinity for oxygen can be carried out in the absence of a solvent. Generally, the treatment is conveniently carried out in a solvent, especially an aprotic solvent. For example, where a Lewis acid or an iodotrialkylsilane is used as the agent having affinity for oxygen, halogenated hydrocarbons such as methylene chloride, chloroform and 1,2-dichloroethane give especially good results. When the trialkylsilyl perfluoroalkanesulfonate is used as the agent, the halogenated hydrocarbons, acetonitrile and orthoformates are preferred as solvents.

The reaction in this embodiment proceeds very smoothly under mild conditions. The treating temperature is about −40°C to about 150°C, preferably about −20°C to about 100°C, more preferably −10°C to about 90°C.

By treating the compound of formula (II) with the agent having affinity for oxygen under these conditions, the compound of formula (I) results. When the agent having affinity for oxygen is a Lewic acid, the product and the agent may sometimes form a complex. In this case, the product may be isolated by adding water to the reaction mixture to decompose the complex and then subjecting it to usual isolating operations.

The compound of formula (I) produced by the process of this invention can be isolated from the

reaction mixture by methods known *per se*, such as extraction, chromatography, distillation, and crystallization.

The compounds of formula (II) used as a starting material in the process of this invention are novel compounds not described in the literature and constitute a part of the present invention.

The various groups in formula (II) are exemplified as follows:

(1) Aryl groups optionally having at least substituent are preferred as the aromatic group Ar. More preferred are groups of the formula $R^6$—$Ar^1$—, wherein $Ar^1$ represents a phenylene or naphthylene group and $R^6$ represents a hydrogen atom, a halogen atom, a lower alkyl group, a lower alkoxy group, a lower haloalkoxy group, a lower alkanoylamino group, an oxo-isoindolinyl group, or a phenyl group. A thienyl group can be cited as another preferred example of the aromatic group Ar.

(2) Lower alkyl groups such as methyl, ethyl, n-propyl, isopropyl, or butyl are preferred as the saturated aliphatic group $R^1$. Thus, a hydrogen atom and lower alkyl groups constitute a preferred class of $R^1$.

(3) Lower alkyl groups are suitable as the alkyl groups represented individually by $R^3$ and $R^4$. Lower alkylene groups such as ethylene, propylene, and trimethylene are suitable as the alkylene group formed by $R^3$ and $R^4$ taken together.

(4) Examples of preferred substituted or unsubstituted alkyl groups represented by $R^5$ include unsubstituted lower alkyl groups, such as methyl, ethyl and butyl; lower haloalkyl groups such as trifluoromethyl; and a d- or l-10-camphoryl. Preferred as the aromatic group $R^5$ are substituted or unsubstituted phenyl groups of the formula

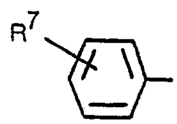

wherein $R^7$ represents a hydrogen atom, a halogen atom, a nitro group, or a lower alkyl group.

Among the compounds of formula (II) provided by this invention, preferred are those of the following compounds

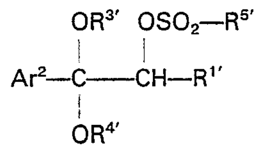

(I-1)

wherein $Ar^2$ represents a group of the formula $R^6$—$Ar^1$— or a thienyl group, $R^{1'}$ represents a hydrogen atom or a lower alkyl group; $R^{3'}$ and $R^{4'}$, independently from each other, represent a lower alkyl group, or taken together, form a lower alkylene group; $R^{5'}$ represents a lower alkyl group, a lower haloalkyl group, a d- or l-10-camphoryl group or the group of the formula

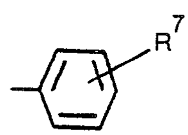 ;

and $Ar^1$, $R^6$ and $R^7$ are as defined above.

In formula (II), Ar and $R^1$ may form a condensed ring together with the carbon atom to which they are bonded. Specific examples of the compounds of formula (II) in which Ar and $R^1$ form a condensed ring are

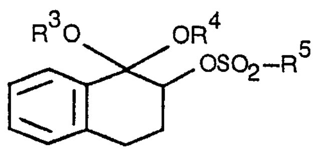

and

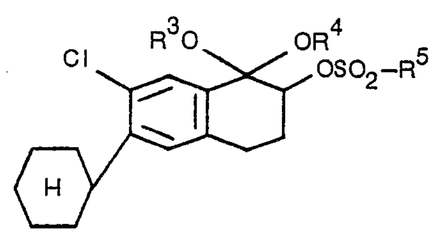 .

Hydrolysis of these compounds or treatment thereof with the agent having affinity for oxygen by the process of this invention respectively yields the following compounds.

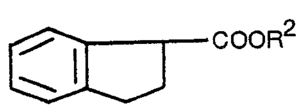

and

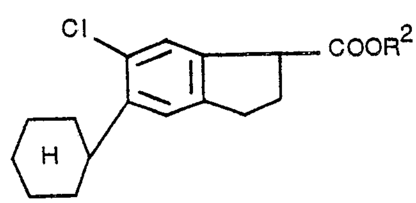 .

The compounds of formula (II) can be synthesized from alpha-haloketones of the general formula

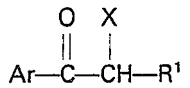

(V)

wherein X represents a halogen atom, and Ar and $R^1$ are as defined above, through the route shown in the following reaction scheme.

Reaction scheme

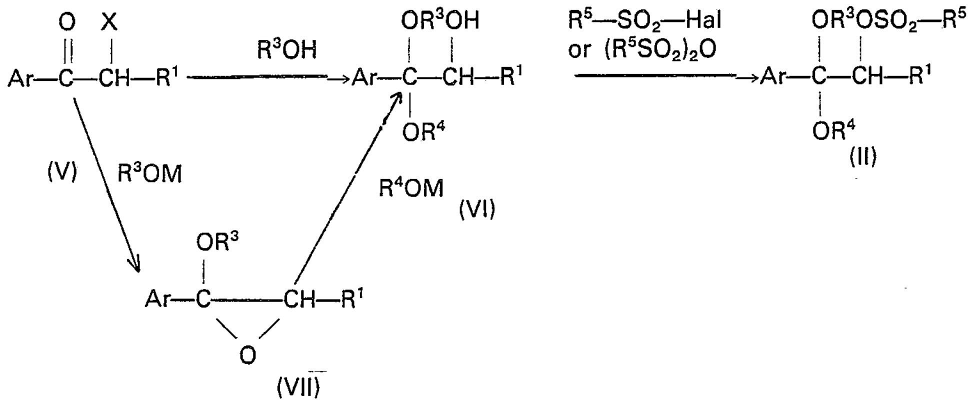


In the above scheme, M represents an alkali metal and Hal represents a halogen atom, especially a chlorine atom, and Ar, $R^1$, $R^3$, $R^4$, $R^5$ and X are as defined hereinabove.

The compound of formula (V) can be produced easily by subjecting a compound of the formula

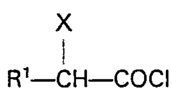

(VIII)

wherein $R^1$ and X are as defined above, to the Friedel-Crafts reaction with a compound of the formula

Ar—H (IX)

wherein Ar is as defined above, or by halogenating a compound of the formula

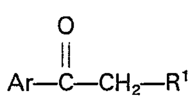

(X)

wherein Ar and $R^1$ are as defined above, in a manner known *per se*.

The route in the Reaction Scheme is described in detail below.

Step (V)→(VI)

This step involves the action of an alkali metal alkoxide ($R^3OM$) on the compound of general formula (V) in the presence of the corresponding alcohol ($R^3OH$) to give an alpha-hydroxyketone acetal of general formula (VI). In the compound of general formula (VI) produced in this step, $R^4$ is the same as $R^3$. Lithium alkoxides, sodium alkoxides and potassium alkoxides can be suitably used as the alkali metal alkoxide. The use of the sodium alkoxides is preferred because of their low cost. The amount of the alkali metal alkoxide is generally at least 1 mole per mole of the compound of formula (V), and the reaction can be completed rapidly if it is used in an amount of 1.5 to 3 moles per mole of the compound of formula (V). The amount of the alcohol to be copresent may be at least 1 mole per mole of the compound of general formula (V). Advantageously, the alcohol is used in excess to make it serve also as a solvent. It is also possible to add an aprotic solvent which does not participate in the reaction, such as diethyl ether, tetrahydrofuran, DMF, or 1,2-dimethoxyethane. The reaction proceeds smoothly at a temperature of about −20°C to about 100°C. For the simplicity of the operation, the reaction is preferably carried out at room temperature to 60°C.

According to another embodiment, this step can be performed by reacting the compound of formula (V) with the alkali metal alkoxide ($R^3OM$) in an aprotic solvent such as diethyl ether, tetrahydrofuran or 1,2-dimethoxyethane to form an epoxy compound of formula (VII), and then reacting it with, an alcohol ($R^4OH$) in the presence of a catalytic amount of an alkali metal alkoxide ($R^4OM$) to obtain an alpha-hydroxyketone acetal (VI) (see Examples 8 and 9). According to this process, a compound of formula (VI) in which $R^3$ differs from $R^4$ can also be produced.

The reaction between the compound of formula (V) and the alkali metal alkoxide ($R^3OM$) can be carried out usually at a temperature of about 0°C to about 60°C using 1 to 3 moles, per mole of the compound of formula (V), of the alkali metal alkoxide. The reaction between the compound of formula (VII) and the alcohol ($R^4OH$) can be performed generally at a temperature of about 0°C to about 100°C by using at least one mole, per mole of the compound of formula (VII), of the alcohol. Preferably the alcohol is used in excess to make it serve also as a solvent.

According to still another embodiment of this step, the alpha-haloketone of general formula (V) is reacted in the presence of a dihydric alcohol such as ethylene glycol, propylene glycol or 1,3-propanediol with a mono-salt of the dihydric alcohol and an alkali metal to give the alpha-hydroxyketone acetal of general formula (VI). This process gives a product corresponding to formula (VI) in which $R^3$ and $R^4$ together form an alkylene group such as ethylene, propylene, or trimethylene (see Example 111).

The reaction can be performed generally at 0°C to 100°C by using at least 1 mole, preferably 1.5 to 3 moles, of the dihydric alcohol mono-salt of alkali metal in the presence of at least 0.5 mole, preferably 2 to 10 moles, of the dihydric alcohol. There can be added an aprotic solvent which does not participate in the reaction such as diethyl ether, tetrahydrofuran or 1,2-dimethoxyethane.

Compounds of formula (VI) obtained in the above step, except those wherein Ar is phenyl or a 4-chlorophenyl group and $R^1$ is a hydrogen atom, are novel compounds and are the subject of EPC Application 84113585.8 (EP—A—151702) divided out of this application.

Typical examples of such compounds (VI) are given below in Examples 3, 9, 12, 17, 65, 70, 72, 74, 78, 82, 85, 88, 92, 97, 100, 105, 108 and 111. The compounds wherein Ar is 4-prenylphenyl, $R^1$ is methyl; Ar is N-methylphenothiazinyl, $R^1$ is hydrogen or methyl; Ar is 1-methylpyrrolyl, $R^1$ is hydrogen or methyl, Ar is 2 - fluoro - 4 - biphenylyl, $R^1$ is methyl; Ar is 2 - acetylamino - 4 - biphenylyl, $R^1$ is methyl; Ar is 4-chlorophenyl, $R^1$ is isopropyl; and Ar is 3 - chloro - 4 - (3 - pyrrolin - 1 - yl)phenyl, $R^1$ is methyl are representative of compounds of formula (VI).

Step (VI)→(II)

This step involves the action of an O-sulfonylating agent of the formula $R^5—SO_2—Hal$ or $(R^5SO_2)_2O$ on the alpha-hydroxyketone acetal of formula (VI) obtained in the above step to form the compound of general formula (II).

Examples of the O-sulfonylating agent include aromatic group substituted sulfonyl halides such as benzenesulfonyl chloride, p-toluenesulfonyl chloride, p-bromobenzenesulfonyl chloride, p-nitrobenzenesulfonyl chloride and naphthalene sulfonyl chloride; and alkanesulfonyl halides or alkanesulfonic anhydrides, such as methanesulfonyl chloride, butanesulfonyl chloride, methanesulfonic anhydride, trifluoromethanesulfonic anhydride, trifluoromethanesulfonyl chloride, d-10-camphorsulfonyl chloride, and 1-10-camphorsulfonyl chloride.

The reaction is desirably carried out under neutral to basic conditions, and from this standpoint, the reaction is advantageously carried out in the presence of at least 1 mole, per mole of the compound of formula (VI), of a tertiary amine such as triethlamine, pyridine, or 4-dimethylaminopyridine. In this way, the reaction can be performed at a relatively low temperature of from about 0°C to room temperature. It is also possible to add an aprotic solvent which does not participate in the reaction, such as methylene chloride or diethyl ether.

As described above, the compound of general formula (II) as the starting material in the process of this invention can be produced in two steps from the alpha-haloketone of general formula (V). It can also be produced by other methods, such as a method which comprises oxidizing a 1 - (aromatic group) - 1 - alkoxy - 1 - alkene of the formula

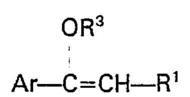
(XI)

wherein Ar, $R^1$, and $R^3$ are as defined hereinabove, to form the epoxy compound of formula (VII), producing the alpha-hydroxyketone acetal of formula (VI) from the epoxy compound as above, and further performing the step (VI)→(II); a process which comprises acetalizing the corresponding alpha-sulfonyloxyketone to the compound of general formula (II); and a method which comprises reducing an alpha-oxoketone acetal of the following formula

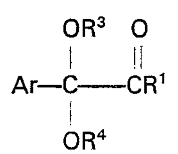
(XII)

wherein Ar, $R^1$, $R^3$, and $R^4$ are as defined above, to form the alpha-hydroxyketone acetal of formula (VI), and subjecting it to the step (VI)→(II).

Thus, the alpha-sulfonyloxyketone acetal of formula (II) as the starting material in the process of this invention can be produced by various easy methods with a lesser number of steps.

The compounds of formula (I) which can be produced by the process of this invention include many compounds useful in such fields as pharmaceuticals and agricultural chemicals. Typical examples of such useful compounds are Ibuprofen, Naproxen, and alpha - [4 - (1 - oxo - 2 - isoindolinyl)phenyl]propionic acid (anti-inflammatory agent, Indoprofen). Typical examples also include alpha - (2 - thienyl)propionic acid, methyl alpha - (4 - acetylaminophenyl)propionate, alpha - [4 - (tert - butyl)phenyl]isovaleric acid, methyl alpha - (4 - alkoxyphenyl)isovalerates, alpha - (4 - biphenylyl)propionic acid, methyl alpha - (4 - difluoromethoxyphenyl)isovalerate, and methyl alpha - (4 - alkoxyphenyl)propionates which are known as important synthetic intermediates for anti-inflammatory agents, and insecticides.

The following Examples illustrate the present invention more specifically.

Example 1

9.81 g of p-toluenesulfonyl chloride was dissolved in 10 ml of anhydrous pyridine, and the mixture was stirred at room temperature. To the solution was added dropwise over 10 minutes 10 ml of an anhydrous pyridine solution of 6.65 g of alpha-hydroxypropiophenone dimethyl acetal, and the mixture was stirred for 48 hours. The reaction mixture was poured onto 200 ml of ice water, and stirred for 2 hours. The resulting precipitate was collected by filtration, washed with water, and dried in vacuum over potassium hydroxide to give 10.91 g of alpha - (p - toluenesulfonyloxy)propiophenone dimethyl acetal as colorless crystals.

Yield: 92%

mp: 62—63°C (from ligroin)

IR (KBr):

1360, 1190, 1175, 1090, 1060, 1040, 910, 710 $cm^{-1}$.

NMR ($CDCl_3$):

δ 1.07 (3H, d, J=6Hz), 2.73 (3H, s), 3.08 (3H, s), 3.15 (3H, s), 4.95 (1H, q, J=6Hz), 7.33 (7H, m), 7.77 (2H, d, J=9Hz).

For $C_{18}H_{22}O_5S$:

| | |
|---|---|
| Calculated: | C, 61.70; H, 6.33; S, 9.15%. |
| Found: | C, 61.72; H, 6.26; S, 9.19%. |

Example 2

19.0 g of p-isobutylpropiophenone was dissolved in a mixture of 15 ml of anhydrous diethyl ether and 5 ml of anhydrous dioxane, and the solution was stirred at room temperature. Bromine (17.6 g) was added dropwise over 25 minutes, and the mixture was stirred for 2.5 hours. Water (50 ml) was added, and the mixture was extracted with 20 ml of diethyl ether three times. The extract was washed with 50 ml of water four times, dried over anhydrous magnesium sulfate, and concentrated under reduced pressure. The residue was dissolved in 30 ml of hot methanol, and cooled to −40°C. The precipitate was collected by filtration, and washed with cold methanol to give 21.88 g of alpha - bromo - p - isobutylpropiophenone as colorless crystals.

Yield: 81%

mp: 63—66°C.

IR (KBr):

2950, 1680, 1608, 1419, 1260, 1250, 1167, 955, 862 $cm^{-1}$.

NMR ($CDCl_3$):

δ 0.91 (6H, d, J=7Hz), 1.87 (3H, d, J=7Hz), 1.8—2.2 (1H, m), 2.52 (2H, d, J=7Hz), 5.25 (1H, q, J=7Hz), 7.22 (2H, d, J=9Hz), 7.92 (2H, d, J=9Hz).

For $C_{13}H_{17}OBr$:

| | |
|---|---|
| Calculated: | C, 58.00; H, 6.37; Br, 29.69% |
| found: | C, 57.80; H, 6.27; Br, 29.42% |

Example 3

Sodium methoxide (1.134 g) was dissolved in 20 ml of anhydrous methanol, and the solution was stirred at room temperature in an atmosphere of argon. To the solution was added dropwise over 30 minutes at room temperature 20 ml of an anhydrous methanol solution of 2.692 g of alpha - bromo - p - isobutylpropiophenone. After the addition, 50 ml of water was added to the reaction mixture, and the mixture was extracted with 20 ml of diethyl ether three times. The extract was washed with 20 ml of water, dried over anhydrous magnesium sulfate and potassium carbonate, and concentrated under reduced pressure in the presence of a small amount of potassium carbonate to give 2.370 g of alpha - hydroxy - p - isobutylpropiophenone dimethyl acetal as a colorless oily substance. The yield was 94%. For elemental analysis, the product was purified by column chromatography (Florisil; methylene chloride).

IR (neat):

3500, 2950, 1460, 1100, 1050, 980, 850, 800, 740 $cm^{-1}$.

NMR ($CDCl_3$):

δ 0.91 (6H, d, J=7Hz), 0.96 (3H, d, J=7Hz), 1.6—2.1 (1H, m), 2.43 (2H, d, J=7Hz), 2.57 (1H, broad s), 3.20 (3H, s), 3.30 (3H, s), 4.06 (1H, q, J=7Hz), 7.08 (2H, d, J=9Hz), 7.33 (2H, d, J=9Hz).

For $C_{15}H_{24}O_3$:

| | |
|---|---|
| Calculated: | C, 71.39; H, 9.59%. |
| Found: | C, 71.39; H, 9.48%. |

Example 4

26.84 g of isobutylbenzene and 29.34 g of aluminum chloride were stirred under ice cooling in 20 ml of carbon disulfide. To the mixture was added dropwise over 30 minutes 40.63 g of alpha-chloropropionyl chloride, and the mixture was stirred under ice cooling for 1.5 hours. It was further stirred for 30 minutes at room temperature. The reaction mixture was poured onto 200 ml of ice water, and 100 ml of conc. hydrochloric acid was added. The mixture was extracted with 100 ml of diethyl ether four times. The extract was washed with 100 ml of water and then four times with 50 ml of a saturated aqueous solution of sodium hydrogen carbonate, dried over anhydrous magnesium sulfate, and concentrated under reduced pressure. The oil residue was dissolved in 40 ml of methanol at an elevated temperature, treated with activated charcoal, and cooled to −40°C. The precipitated crystals were collected by filtration, washed with cooled methanol to give 17.83 g of alpha - chloro - p - isobutylpropiophenone as colorless crystals having a melting point of 51 to 53°C.

Yield: 40%

IR (KBr):

2950, 1690, 1608, 1419, 1357, 1266, 1180, 956, 861 $cm^{-1}$.

NMR ($CDCl_3$):

δ 0.91 (6H, d, J=7Hz), 1.71 (3H, d, J=7Hz), 1.5—2.2 (1H, m), 2.52 (2H, d, J=7Hz), 5.20 (1H, q, J=7Hz), 7.23 (2H, d, J=9Hz), 7.91 (2H, d, J=9Hz).

For $C_{13}H_{17}OCl$:

| | |
|---|---|
| Calculated: | C, 69.47; H, 7.63; Cl, 15.78%. |
| Found: | C, 69.56; H, 7.44; Cl, 15.76%. |

Example 5

Sodium methoxide (0.979 g) was dissolved in 10 ml of anhydrous methanol, and the solution was stirred at room temperature in an argon atmosphere. To the solution was added dropwise over 20 minutes 10 ml of an anhydrous methanol solution of 2.036 g of alpha - chloro - p - isobutylpropiophenone and the mixture was further stirred at room temperature for 4 hours. Usual work-up as in Example 4 gave 2.158 g (yield 95%) of alpha - hydroxy - p - isobutylpropiophenone dimethyl acetal as a pale yellow oil. Yield 95%.

Example 6

In a similar operation as in Example 1, alpha - (p - toluenesulfonyloxy) - p - isobutylpropiophenone dimethyl acetal was prepared as colorless crystals from alpha - hydroxy - p - isobutylpropiophenone dimethyl acetal.

Yield: 89%.

mp: 64—65°C (from hexane).

IR (KBr):

2960, 1360, 1185, 1090, 1050, 930, 915, 780 $cm^{-1}$.

NMR ($CDCl_3$):

δ 0.88 (6H, d, J=7Hz), 1.09 (3H, d, J=7Hz), 1.5—2.1 (1H, m), 2.40 (3H, s), 2.42 (2H, d, J=7Hz), 3.07 (3H, s), 3.15 (3H, s), 4.93 (1H, q, J=7Hz), 6.9—7.4 (6H, m), 7.72 (2H, d, J=9Hz).

For $C_{22}H_{30}O_5S$:

| | |
|---|---|
| Calculated: | C, 64.99; H, 7.44; S, 7.89%. |
| Found: | C, 64.78; H, 7.26; S, 7.93%. |

Example 7

Methanesulfonyl chloride (2.94 g) was dissolved in 5 ml of anhydrous pyridine, and the solution was stirred under ice cooling. To the solution was added dropwise over 15 minutes 10 ml of an anhydrous pyridine solution of 3.23 g of alpha - hydroxy - p - isobutylpropiophenone dimethyl acetal. The mixture was stirred under ice cooling for 1 hour, and at room temperature for 30 minutes. Water (30 ml) was added, and the mixture was stirred for 1 hour. The mixture was extracted with 10 ml of methylene chloride three times. The extract was washed with 10 ml of water three times, dried over anhydrous magnesium sulfate, and concentrated in the presence of a small amount of anhydrous potassium carbonate. The oily residue was purified by column chromatography (Florisil; methylene chloride) to give 3.347 g of alpha - (methanesulfonyloxy) - p - isobutylpropiophenone dimethyl acetal as a colorless oil.

Yield: 79%.

IR (neat):

2960, 1350, 1175, 1095, 1065, 1040, 915 $cm^{-1}$.

NMR ($CDCl_3$):

δ 0.88 (6H, d, J=7Hz), 1.16 (3H, d, J=7Hz), 1.6—2.1 (1H, m), 2.44 (2H, d, J=7Hz), 3.06 (3H, s), 3.18 (3H, s), 3.26 (3H, s), 4.95 (1H, q, J=7Hz), 7.10 (2H, d, J=8Hz), 7.32 (2H, d, J=8Hz).

For $C_{16}H_{26}O_5S$:

| | |
|---|---|
| Calculated: | C, 58.16; H, 7.93; S, 9.69%. |
| Found: | C, 58.06; H, 7.80; S, 9.60%. |

Example 8

Sodium methoxide (9.20 g) was suspended in 10 ml of anhydrous diethyl ether, and in an atmosphere of argon, the solution was stirred under ice cooling. To the solution was added dropwise over 40 minutes 80 ml of an anhydrous diethyl ether solution of 15.0 g of alpha - bromo - p - methoxypropiophenone. After the addition, the insoluble matter was separated by filtration in a stream of argon. The filtrate was concentrated under reduced pressure to give 7.88 g of 1 - (4 - methoxyphenyl) - 1 - methoxy - 1,2 - epoxypropane as a pale red oil.

Yield: 68%.

NMR ($CDCl_3$):

δ 1.00 (3H, d, J=6Hz), 3.23 (3H, s), 3.52 (1H, q, J=6Hz), 3.82 (3H, s), 6.92 (2H, d, J=7Hz), 7.38 (2H, d, J=7Hz).

Example 9

7.88 g of 1 - (4 - methoxyphenyl) - 1 - methoxy - 1,2 - epoxypropane and 200 mg of sodium methoxide were stirred in 30 ml of anhydrous methanol at room temperature for 15 hours. Water (50 ml) was added to the reaction solution, and the mixture was extracted with 20 ml of diethyl ether three times. The extracts were dried over anhydrous magnesium sulfate and anhydrous potassium carbonate, and concentrated under reduced pressure in the presence of a small amount of anhydrous potassium carbonate to give 8.26 g of alpha - hydroxy - p - methoxypropiophenone dimethyl acetal as a colorless oil. For elemental analysis, this produce was purified by column chromatography (Florisil; methylene chloride).

Yield: 61%.

IR (neat):

3520, 1050 $cm^{-1}$.

NMR ($CDCl_3$):

δ 0.94 (3H, d, J=7Hz), 2.35 (1H, d, J=4Hz), 3.20 (3H, s), 3.33 (3H, s), 3.78 (3H, s), 4.05 (1H, dq, J=4 and 7Hz), 6.18 (2H, d, J=9Hz), 7.35 (2H, d, J=9Hz).

For $C_{12}H_{18}O_4$:

| | |
|---|---|
| Calculated: | C, 63.70; H, 8.02%. |
| Found: | C, 63.98; H, 8.08%. |

Example 10

2.67 g of p-toluenesulfonyl chloride was dissolved in 5 ml of anhydrous pyridine, and the solution was stirred at room temperature. To the solution was added dropwise over 10 minutes 5 ml of an anhydrous pyridine solution of 1.58 g of alpha - hydroxy - p - methoxypropiophenone dimethyl acetal. The mixture was stirred for 72 hours. Ice water (about 20 g) was added, and the mixture was extracted with 20 ml of diethyl ether three times. The extracts were washed with 20 ml of water, dried over anhydrous magnesium sulfate, and concentrated under reduced pressure in the presence of a small amount of anhydrous potassium carbonate to give 2.12 g of alpha - (p - toluenesulfonyloxy) - p - methoxypropiophenone dimethyl acetal as a pale yellow oil.

Yield: 80%.

IR (neat):

1600, 1510, 1350, 1245, 1170, 1090, 1055, 1035, 905 $cm^{-1}$.

NMR ($CDCl_3$):

δ 1.07 (3H, d, J=7Hz), 2.40 (3H, s), 3.07 (3H, s), 3.12 (3H, s), 3.75 (3H, s), 4.92 (1H, q, J=7Hz), 6.7—7.0 (2H, m), 7.1—7.4 (4H, m), 7.73 (2H, d, J=8Hz).

Example 11

In a similar operation as in Example 2, alpha - bromo - p - (tert - butyl)isovalerophenone was prepared as a colorless liquid from p - (tert - butyl)isovalerophenone.

Yield: 72%.

bp: 128—140°C/1 torr.

IR (neat):

2960, 1680, 1600 $cm^{-1}$.

NMR ($CDCl_3$):

δ 1.00 (3H, d, J=7Hz), 1.20 (3H, d, J=7Hz), 1.35 (9H, s), 2.2—2.7 (1H, m), 4.90 (1H, d, J=9Hz), 7.47 (2H, d, J=10Hz), 7.90 (2H, d, J=10Hz).

For $C_{15}H_{21}OBr$:

| | |
|---|---|
| Calculated: | C, 60.61; H, 7.12; Br, 26.89%. |
| Found: | C, 60.71; H, 7.11; Br, 26.58%. |

Example 12

Sodium methoxide (1.62 g) was dissolved in 20 ml of anhydrous methanol, and the solution was stirred at room temperature under an atmosphere of argon. To the resulting solution was added 20 ml of an anhydrous methanol solution of 2.64 g of alpha - bromo - p - (tert - butyl)isovalerophenone over 15 minutes and the mixture was stirred at room temperature for 18 hours.

Usual work-up as in Example 3 gave 2.44 g of alpha - hydroxy - p - (tert - butyl)isovalerophenone dimethyl acetal as a pale yellow oil.

Yield: 98%.

IR (neat):

2950, 1110, 1040 $cm^{-1}$.

NMR ($CDCl_3$):

δ 0.70 (3H, d, J=7Hz), 0.90 (3H, d, J=7Hz), 1.32 (9H, s), 1.0—1.6 (1H, m), 2.45 (1H, broad s), 3.22 (3H, s), 3.25 (3H, s), 3.67 (1H, d, J=6Hz), 7.33 (4H, s).

Example 13

Methanesulfonyl chloride (1.90 g) was dissolved in 5 ml of anhydrous pyridine, and the solution was stirred at room temperature, and to the solution was added dropwise over 10 minutes 5 ml of an anhydrous pyridine solution of 2.34 g of alpha - hydroxy - p - (tert - butyl)isovalerophenone dimethyl acetal. The mixture was stirred at this temperature further for 24 hours. Usual work-up and separation as in Example 7 gave 1.63 g of alpha - (methanesulfonyloxy) - p - (tert - butyl)isovalerophenone dimethyl acetal as a colorless oil (upon standing at room temperature, this compound crystallized).

Yield: 55%.

mp: 80—86°C.

IR (neat):

2960, 1357, 1176, 1060, 955 $cm^{-1}$.

NMR ($CDCl_3$):

δ 0.70 (3H, d, J=7Hz), 0.90 (3H, d, J=7Hz), 1.32 (9H, s), 1.4—1.9 (1H, m), 3.17 (3H, s), 3.20 (3H, s), 3.26 (3H, s), 4.76 (1H, d, J=4Hz), 7.37 (4H, s).

For $C_{18}H_{30}O_5S$:

| | |
|---|---|
| Calculated | C, 60.30; H, 8.44; S, 8.95% |
| Found: | C, 60.07; H, 8.23; S, 9.07% |

Example 14

Benzene (100 ml) was added to a mixture of 7.12 g of alpha-acetoxyacetophenone, 7.44 g of ethylene glycol and 0.200 g of p-toluenesulfonic acid monohydrate, and the mixture was heated under reflux. Water generated was removed by means of a Dean-Stark device secured to the reactor. After refluxing for 21 hours, 4.00 g of ethylene glycol was added. The mixture was further heated under reflux for 6 hours. Water generated was distilled off. After cooling, 30 ml of a saturated aqueous solution of sodium hydrogen carbonate and 20 ml of water were successively added, and the mixture was extracted with 30 ml of diethyl ether twice. The extracts were washed with 10 ml of water, dried over anhydrous magnesium sulfate and concentrated under reduced pressure to give 7.874 g of a residue. The residue was determined by NMR and GLC to be a mixture of alpha-acetoxyacetophenone ethylene acetal and alpha-hydroxyacetophenone ethylene acetal. The mixture was dissolved in 20 ml of methanol, and 0.300 g of potassium carbonate was added. The mixture was stirred at room temperature for 4 hours. Water (200 ml) was added, and the precipitated colorless crystals were collected by filtration and dried overnight in vacuum on potassium hydroxide to give 4.79 g of alpha-hydroxyacetophenone ethylene acetal.

Yield: 67%.

NMR ($CDCl_3$):

δ 2.10 (1H, broad s), 3.70 (2H, broad s), 3.7—4.0 (2H, m), 4.0—4.2 (2H, m), 7.2—7.6 (5H, m).

Example 15

Pyridine (5 ml) was added to 0.900 g of alpha-hydroxyacetophenone ethylene acetal, and the mixture was stirred under ice cooling. To the mixture was added 1.08 g of p-toluenesulfonyl chloride, and the mixture was stirred for 2 hours under ice cooling and then for 1 hour at room temperature. The reaction mixture was again cooled with ice. Water (10 ml was added, and the mixture was stirred for 1 hour and extracted with 20 ml of diethyl ether three times. The extracts were washed with 10 ml of water twice, dried over anhydrous magnesium sulfate, and concentrated under reduced pressure to give 1.584 g of a crystalline residue. The residue was washed with a small amount of hexane to give 1.330 g of alpha - (p - toluenesulfonyloxy)acetophenone ethylene acetal as colorless crystals.

Yield: 80%.

NMR ($CDCl_3$):

δ 2.40 (3H, s), 3.7—3.9 (2H, m), 3.9—4.1 (2H, m), 4.11 (2H, s), 7.1—7.5 (5H, m), 7.55 (2H, d, J=8Hz).

Example 16

In a similar operation in Example 15, alpha(p-bromobenzenesulfonyloxy)acetophenone ethylene acetal was prepared as colorless crystals from alpha-hydroxyacetophenone ethylene acetal.

Yield: 86%.
NMR ($CDCl_3$):
δ 3.7—3.9 (2H, m), 3.9—4.1 (2H, m), 4.16 (2H, s), 7.2—7.5 (5H, m), 7.63 (4H, s).

Example 17

Metallic sodium (0.46 g) was dissolved in 10 ml of anhydrous methanol, and the solution was stirred at room temperature. To the solution was added dropwise over 10 minutes 10 ml of an anhydrous methanol solution of 2.19 g of 1 - (2 - thienyl) - 2 - bromo - 1 - propanone and the mixture was stirred further for 3.5 hours at room temperature. Usual work-up as in Example 3 gave 1.900 g of 1 - (2 - thienyl) - 2 - hydroxy - 1 - propanone dimethyl acetal as a colorless oil.

Yield: 94%.
IR (neat):
3450, 1110, 1055, 980, 855, 710 $cm^{-1}$.
NMR ($CDCl_3$):
δ 1.05 (3H, d, J=7Hz), 2.54 (1H, broad s), 3.26 (3H, s), 3.30 (3H, s), 4.16 (1H, broad q, J=7Hz), 6.9—7.2 (2H, m), 7.2—7.4 (1H, m).

Example 18

0.606 g of 1 - (2 - thienyl) - 2 - hydroxy - 1 - propanone dimethyl acetal was dissolved in 5 ml of pyridine, and the solution was stirred under ice cooling. To the solution was added over 1 minute 0.30 ml of methanesulfonyl chloride, and the mixture was stirred for 5 minutes under ice cooling and then for 5 hours at room temperature. Water (30 ml) was added, and the mixture was stirred for 30 minutes at room temperature, and extracted with 30 ml of diethyl ether three times. The extracts were washed with 10 ml of water twice, dried over anhydrous magnesium sulfate and anhydrous potassium carbonate, and concentrated under reduced pressure. The residue was purified by column chromatography (Florisil, methylene chloride) to give 0.788 g of 1 - (2 - thienyl) - 2 - (methanesulfonyloxy) - 1 - propanone dimethyl acetal as a colorless oil.

Yield: 94%.
IR (neat):
1360, 1180, 1120, 1055, 990, 975, 930, 905, 855, 810, 715, 540, 525 $cm^{-1}$.
NMR ($CDCl_3$):
δ 1.27 (3H, d, J=7Hz), 3.09 (3H, s), 3.27 (6H, s), 5.02 (1H, q, J=7Hz), 6.9—7.1 (2H, m), 7.2—7.4 (1H, m).
For $C_{10}H_{16}O_5S_2$:

| | |
|---|---|
| Calculated: | C, 42.84; H, 5.75; S, 22.87%. |
| Found: | C, 42.64; H, 5.64; S, 22.90%. |

Example 19

Benzenesulfonyl chloride (2.01 g) was dissolved in 10 ml of anhydrous pyridine, and the solution was stirred at room temperature. To the solution was added dropwise over 15 minutes 10 ml of an anhydrous pyridine solution of 1.93 g of alpha - hydroxy - p - isobutylpropiophenone dimethyl acetal, and the mixture was stirred for 72 hours. By a similar work-up to Example 1, 2.62 g of alpha - (benzenesulfonyloxy) - p - isobutylpropiophenone dimethyl acetal was obtained as colorless crystals.

Yield: 88%.
mp: 79—82°C.
IR (KBr):
2950, 1362, 1195, 1097, 1043, 990, 921, 813, 594 $cm^{-1}$.
NMR ($CDCl_3$):
δ 0.86 (6H, d, J=7Hz), 1.08 (3H, d, J=7Hz), 1.6—2.1 (1H, m), 2.41 (2H, d, J=7Hz), 3.02 (3H, s), 3.10 (3H, s), 4.94 (1H, q, J=7Hz), 7.04 (2H, d, J=9Hz), 7.19 (2H, d, J=9Hz), 7.36—7.70 (3H, m), 7.84—8.02 (2H, m).

Example 20

1.91 g of p-toluenesulfonyl chloride was dissolved in 10 ml of anhydrous pyridine, and the solution was stirred at room temperature. To the solution was added dropwise over 10 minutes 10 ml of an anhydrous pyridine solution of 1.13 g of alpha-hydroxypropiophenone diethyl acetal, and the mixture was stirred for 72 hours. Usual work-up and purification by column chromatography (Florisil, methylene chloride) gave 1.16 g of alpha - (p - toluenesulfonyloxy) - propiophenone diethyl acetal as a colorless oil.

Yield: 85%.
IR (neat):
2980, 1360, 1192, 1178, 1087, 1055, 1042, 920 $cm^{-1}$.
NMR ($CDCl_3$):
δ 0.8—1.3 (9H, m), 2.38 (3H, s), 3.1—3.6 (4H, m), 4.89 (1H, q, J=7Hz), 7.1—7.5 (7H, m), 7.80 (2H, d, J=9Hz).

For $C_{20}H_{26}O_5S$:
Calculated: C, 63.46; H, 6.93; S, 8.47%.
Found: C, 63.54; H, 6.90; S, 8.35%.

Example 21

0.526 g of alpha - (p - toluenesulfonyloxy)propiophenone dimethyl acetal and 0.150 g of calcium carbonate was heated under reflux for 72 hours in 10 ml of a mixture of water and methanol (3:7 by weight). Water (10 ml) was added, and the mixture was extracted with 10 ml of diethyl ether three times. The extracts were washed with 10 ml of water, and dried over anhydrous magnesium sulfate. The product was quantitatively analyzed by gas chromatography (internal standard method) and found to contain 0.162 g of methyl alpha-phenylpropionate. Yield 66%.

Example 22

1.75 g of alpha - (p - toluenesulfonyloxypropiophenone dimethyl acetal and 0.500 g of calcium carbonate were heated under reflux for 72 hours in 33 ml of a mixture of water and methanol (3:7 by weight). Water (50 ml) was added, and the mixture was extracted with 30 ml of diethyl ether three times. The extracts were washed with 30 ml of water, and concentrated under reduced pressure to about 5 ml. Methanol (12 ml), 5 ml of water, and 7 ml of a 10% aqueous solution of potassium hydroxide were added to the residue, and the mixture was heated under reflux for 5 hours. Water (30 ml) was added, and the mixture was washed with 20 ml of diethyl ether five times. The aqueous layer was adjusted to pH 1 with conc. hydrochloric acid, and extracted with 20 ml of diethyl ether four times. The extracts were washed with 20 ml of water, dried over anhydrous magnesium sulfate, and concentrated under reduced pressure to give 0.358 g of alpha-phenylpropionic acid as a colorless oil. Yield 48%. This product completely agreed with an authentic sample in IR and NMR spectral data.

Examples 23 to 35

0.526 g of alpha - (p - toluenesulfonyloxy)propiophenone dimethyl acetal and an equimolar amount of each of the various bases shown in Table 1 were heated on an oil bath in 10 ml of each of the various solvents shown in Table 1. Water (10 ml) was added, and the mixture was extracted with 10 ml of diethyl ether three times. The extracts were washed with 10 ml of water and dried over anhydrous magnesium sulfate.

The product was analyzed for methyl alpha-phenylpropionate by gas chromatography in the same way as in Example 21.

The results are shown in Table 1.

TABLE 1

| Example | Solvent (mixing ratio) | Base | Bath temperature (°C) | Reaction time (hours) | Yield of methyl alpha-phenylpropionate (%) |
|---|---|---|---|---|---|
| 23 | Dioxane:water (9:1) | $CH_3COOK$ | 110 | 216 | 54.3 |
| 24 | Dioxane:water (6:4) | $CaCO_3$ | 110 | 64 | 56.6 |
| 25 | DMF:water (9:1) | $CH_3COOK$ | 150 | 14 | 40.3 |
| 26 | DMF:water (6:4) | $CH_3COOK$ | 150 | 14 | 55.6 |
| 27 | DMF:water (6:4) | $CaCO_3$ | 100 | 94 | 54.6 |
| 28 | DMF:water (6:4) | $CaCO_3$ | 100 | 72 | 54.4 |
| 29 | DMSO:water (6:4) | $CaCO_3$ | 100 | 94 | 54.3 |
| 30 | DMSO:water (4:6) | $CaCO_3$ | 100 | 72 | 58.5 |
| 31 | Anhydrous methanol | — | 150(*) | 7 | 34.2 |
| 32 | Methanol:water (7:3) | $CH_3COOK$ | 110 | 72 | 60.8 |
| 33 | Methanol:water (6:4) | $CaCO_3$ | 110 | 64 | 63.0 |
| 34 | Methanol:water (4:6) | $CaCO_3$ | 110 | 72 | 33.3 |
| 35 | Acetic acid | $CH_3COOK$ | 130 | 14 | 39.0 |

(*) Heated in a 30 ml sealed tube.

Example 36

0.410 g of alpha - (p - toluenesulfonyloxy) - p - isobutylpropiophenone dimethyl acetal and 0.100 g of calcium carbonate were heated under reflux in a mixture of water and methanol (3:7 by weight) for 22 hours. Water (15 ml) was added, and the mixture was extracted with 10 ml of diethyl ether three times. The extracts were washed with 10 ml of water, dried over anhydrous magnesium sulfate, and concentrated under reduced pressure. The oily residue was purified by column chromatography (silica gel, methylene chloride) to give 0.180 g of methyl alpha - (4 - isobutylphenyl)propionate as a colorless oil. Yield 81%. This product completely agreed with an authentic sample in IR and NMR spectral data.

Example 37

0.910 g of alpha - (p - toluenesulfonyloxy) - p - isobutylpropiophenone dimethyl acetal and 1.25 g of potassium carbonate were heated for 20 hours under reflux in 26 ml of a mixture of water and methanol (3:7 by weight). Water (30 ml) was added, and the mixture was washed with 10 ml of methylene chloride four times. The aqueous layer was adjusted to pH 2 with dilute hydrochloric acid, and extracted with 20 ml of methylene chloride four times. The extracts were washed with 30 ml of water, dried over anhydrous magnesium sulfate to give 0.335 g of alpha - (4 - isobutylphenyl)propionic acid as colorless crystals having a melting point of 71 to 75°C. Yield 73%. This product completely agreed with an authentic sample in IR and NMR spectral data.

Example 38

0.991 g of alpha - (methanesulfonyloxy) - p - isobutylpropiophenone dimethyl acetal and 0.300 g of calcium carbonate were heated under reflux for 72 hours in 10 ml of a mixture of water and methanol (3:7 by weight). Usual work-up followed by alkaline hydrolysis of the crude product as in Example 22 gave 0.519 g of alpha - (4 - isobutylphenyl)propionic acid as colorless crystals having a melting point of 73 to 75°C. Yield 84%. This product completely agreed with an authentic sample in IR and NMR spectral data.

Example 39

2.12 g of alpha - (p - toluenesulfonyloxy) - p - methoxypropiophenone dimethyl acetal and 0.560 g of calcium carbonate were heated under reflux for 13 hours in 20 ml of a mixture of water and methanol (3:7 by weight). Usual work-up followed by alkaline hydrolysis of the crude product as in Example 22 gave 0.714 g of alpha - (4 - methoxyphenyl)propionic acid as pale yellow crystals having a melting point of 47 to 50°C. Yield 57%.

Example 40

0.735 g of alpha - (methanesulfonyloxy) - p - (tert - butyl)isovalerophenone dimethyl acetal and 0.200 g of calcium carbonate were heated under reflux for 72 hours in 10 ml of a mixture of water and methanol (3:7 by weight). Usual work-up as in Example 36 and purification by column chromatography (silica gel, methylene chloride) to give methyl alpha - [4 - (tert - butyl)phenyl]isovalerate as a colorless oil.

IR (neat):

2965, 1745, 1165, 1025 $cm^{-1}$.

NMR ($CDCl_3$):

δ 0.71 (3H, d, J=7Hz), 1.02 (3H, d, J=7Hz), 1.32 (9H, s), 2.1—2.6 (1H, m), 3.10 (1H, d, J=11Hz), 3.60 (3H, s), 7.1—7.4 (4H, m).

For $C_{16}H_{24}O_2$:

| | |
|---|---|
| Calculated: | C, 77.37; H, 9.74%. |
| Found: | C, 77.26; H, 9.62%. |

Example 41

0.668 g of alpha - (p - toluenesulfonyloxy)acetophenone ethylene acetal was dissolved in 8 ml of 1,3 - dimethyl - 2 - imidazolidinone (DMI), and 200 mg of calcium carbonate and 0.2 ml of water were added. The mixture was heated at 180°C for 22 hours with stirring. After cooling, 30 ml of water was added, and the mixture was extracted with 20 ml of diethyl ether three times. The extracts were washed with 10 ml of water twice, and dried over anhydrous magnesium sulfate. The aqueous layers were combined, and 4 ml of conc. hydrochloric acid was added. The mixture was extracted with 20 ml of diethyl ether three times. The extracts were washed with 10 ml of water twice and dried over anhydrous magnesium sulfate. The residues left after concentrating under reduced pressure the extracts obtained in neutrality and acidity respectively were combined, and 400 ml of potassium hydroxide was added. The mixture was heated under reflux for 16 hours in a mixture of 6 ml of methanol and 2 ml of water. After cooling, 30 ml of water was added, and the mixture was washed with 20 ml of diethyl ether three times. The aqueous layer was acidified to a pH of about 2 by adding about 7 ml of 3.5% hydrochloric acid and extracted with 20 ml of diethyl ether three times. The extracts were washed with 10 ml of water, dried over anhydrous magnesium sulfate, and concentrated under reduced pressure to give 92 mg of a residue. This residue was found to be a mixture of benzoic acid and phenylacetic acid by its NMR and GLC of its methyl ester resulting from treatment with diazomethane. NMR spectral data showed that it contained 65 mg (yield 27%) of benzoic acid and 27 mg (yield 10%) of phenylacetic acid.

Example 42

0.798 g of alpha - (p - bromobenzenesulfonyloxy)acetophenoneethylene acetal was dissolved in 8 ml of DMI, and 200 mg of calcium carbonate and 0.5 ml of water were added. The mixture was heated at 160°C for 18 hours with stirring. In the same way as in Example 41, the reaction mixture was worked up and the crude product was subjected to alkaline hydrolysis to yield 84 mg of a semisolid. The semisolid was found by NMR to be a mixture of benzoic acid and phenylacetic acid (7.0:4.5 by mole). Accordingly, the mixture was determined to contain 49 mg (yield 20%) of benzoic acid and 35 mg (yield 13%) of phenylacetic acid.

Example 43

0.560 g of 1 - (2 - thienyl) - 2 - (methanesulfonyloxy) - 1 - propanone dimethyl acetal and 0.200 g of calcium carbonate were heated under reflux for 42 hours in a mixture of water and methanol (3:7 by weight). Water (30 ml) was added, and the mixture was extracted with 30 ml of diethyl ether three times. The extracts were washed with 10 ml of water twice, dried over anhydrous magnesium sulfate, and concentrated under reduced pressure to give 316 mg of methyl alpha - (2 - thienyl)propionate as a colorless oil. This product completely agreed with an authentic sample in IR and NMR spectral data. Yield 93% (in crude form).

The crude oil was subjected to simple distillation at 130°C (bath temperature) and 17 torr to give 279 mg of a pure product. Yield 82% (after purification).

The aqueous layers obtained by extraction were combined, and 6 ml of conc. hydrochloric acid was added. The mixture was extracted with 15 ml of diethyl ether three times. The extracts were washed with 10 ml of water twice, dried over anhydrous magnesium sulfate, and concentrated under reduced pressure to give 20 mg of alpha - (2 - thienyl)propionic acid as a colorless oil. Yield 6%. This product completely agreed with an authentic sample in IR and NMR spectral data.

Example 44

0.565 g of 1 - (2 - thienyl) - 2 - methanesulfonyloxy) - 1 - propanone dimethyl acetal and 0.202 g of calcium carbonate were heated under reflux for 21 hours in 6 ml of a mixture of water and methanol (3:7 by weight). To the reaction mixture was added 0.420 g of sodium hydroxide, and the mixture was stirred at room temperature for 15 hours and then heated under reflux for 2 hours. Water (30 ml) was added, and the mixture was washed with 20 ml of diethyl ether. Conc. hydrochloric acid (4 ml) was added to the aqueous layer, and the mixture was extracted with 20 ml of diethyl ether three times. The extracts were washed with 10 ml of water, dried over anhydrous magnesium sulfate, and concentrated under reduced pressure to give 0.299 g of alpha - (2 - thienyl)propionic acid as a colorless oil. Yield 95%.

Example 45

Sodium hydroxide (0.420 g) was dissolved in 6 ml of a mixture of water and methanol (3:7 by weight), and 0.574 g of 1 - (2 - thienyl) - 2 - methanesulfonyloxy) - 1 - propanone dimethyl acetal was added. The mixture was.heated under reflux for 21 hours. Water (40 ml) was added, and the mixture was washed with 20 ml of diethyl ether. Conc. hydrochloric acid (3 ml) was added to the aqueous layer, and the mixture was extracted with 20 ml of diethyl ether three times. The extracts were washed with 5 ml of water, dried over anhydrous magnesium sulfate, and concentrated under reduced pressure to give 0.291 g of alpha - (2 - thienyl)propionic acid as a colorless oil. Yield 91%.

Example 46

0.674 g of 1 - (2 - thienyl) - 2 - (p - toluenesulfonyloxy) - 1 - propanone dimethyl acetal and 0.200 g of calcium carbonate were heated under reflux for 12 hours in 10 ml of a mixture of water and methanol (3:7 by weight). Usual work-up as in Example 36 gave an oily crude product, which was subjected to simple distillation at 110 to 120°C (bath temperature) and 17 torr to give 0.253 g of methyl alpha - (2 - thienyl)propionate as a colorless oil. Yield 79%.

Example 47

0.684 g of 1 - (2 - thienyl) - 2 - benzenesulfonyloxy) - 1 - propanone dimethyl acetal and 0.200 g of calcium carbonate were heated under reflux for 8 hours in 10 ml of a mixture of water and methanol (3:7 by weight). Usual work-up as in Example 36 gave an oily crude product, which was subjected to simple distillation at 110°C (bath temperature) and 15 torr to give 0.277 g of methyl alpha - (2 - thienyl)propionate as a colorless oil. Yield 81%.

Example 48

0.299 g of alpha - (methanesulfonyloxy) - p - acetylaminopropiophenone dimethyl acetal and 0.090 g of calcium carbonate were heated under reflux for 21 hours in 3 ml of a mixture of water and methanol (3:7 by weight). Water (30 ml) was added, and the mixture was extracted with 20 ml of methylene chloride three times. The extracts were washed with 10 ml of water, dried over anhydrous magnesium sulfate, and concentrated under reduced pressure to give a crystalline residue. The residue was purified by column chromatography (silica gel, methylene chloride-diethyl ether) to give 0.186 g of methyl alpha - (4 - acetylaminophenyl)propionate as colorless crystals.

Yield: 93%.

mp: 107—109°C (from ethanol-hexane).

IR (KBr):

1740, 1665, 1610, 1555, 1515, 1415, 1330, 1160, 860, 840 $cm^{-1}$.

NMR ($CDCl_3$):

δ 1.46 (3H, d, J=7Hz), 2.11 (3H, s), 3.64 (3H, s), 3.67 (1H, q, J=7Hz), 7.17 (2H, d, J=8Hz), 7.53 (2H, d, J=8Hz), 8.17 (1H, broads).

For $C_{12}H_{15}NO_3$:

| | |
|---|---|
| Calculated: | C, 65.14; H, 6.83; N, 6.33%. |
| Found: | C, 64.94; H, 7.00; N, 6.27%. |

Example 49

0.329 g of alpha - (benzenesulfonyloxy) - p - isobutylpropiophenone dimethyl acetal and 0.10 g of calcium carbonate were heated under reflux for 10 hours in 10 ml of a mixture of water and methanol (3:7 by weight). Similar work-up to Example 48 and purification by column chromatography (silica gel, methylene chloride) gave 0.181 g of methyl alpha - (4 - isobutylphenyl)propionate as a colorless oil. Yield 82%.

Example 50

0.392 g of alpha - (benzenesulfonyloxy) - p - isobutylpropiophenone dimethyl acetal was heated in 4

ml of a mixture of water and pyridine (1:3 by weight) over an oil bath at 100°C for 19 hours. Similar work-up to Example 48 and purification by column chromatography (silica gel, methylene chloride) gave 0.164 g of methyl alpha - (4 - isobutylphenyl)propionate as a colorless oil. Yield 74%.

Example 51

0.392 g of alpha - (benzenesulfonyloxy) - p - isobutylpropiophenone dimethyl acetal was mixed with 10 ml of a mixture of water and methanol (3:7 by weight) and 2 ml of a 1% aqueous solution of potassium hydroxide, and the mixture was heated under reflux for 9 hours. Water (30 ml) was added, and the mixture was washed with 15 ml of methylene chloride four times. The aqueous layer was adjusted to pH less than 1 with conc. hydrochloric acid, and extracted with 15 ml of methylene chloride four times. The extracts were dried over anhydrous magnesium sulfate, and concentrated under reduced pressure to give 0.138 g of alpha - (4 - isobutylphenyl)propionic acid as colorless crystals having a melting point of 73 to 75°C. Yield 67%.

Example 52

0.150 g of alpha - (p - toluenesulfonyloxy)propiophenone diethyl acetal and 0.05 g of calcium carbonate were heated under reflux for 72 hours in 10 ml of a mixture of water and methanol (3:7 by weight). Water (20 ml) was added, and the mixture was extracted with 5 ml of diethyl ether four times, and dried over anhydrous magnesium sulfate. The product was quantitatively analyzed by gas chromatography (internal standard method), and found to contain 23 mg of ethyl alpha-phenylpropionate. Yield 32%.

Example 53

0.978 g of alpha - (methanesulfonyloxy) - p - fluoropropiophenone dimethyl acetal and 0.33 g of calcium carbonate were heated under reflux for 24 hours in 15 ml of mixture of water and methanol (3:7 by weight). By the same work-up and purification as in example 49, 0.454 g of methyl alpha - (4 - fluorophenyl)propionate was obtained as a colorless oil.

Yield: 76%.

IR (neat):

1744, 1607, 1513, 1460, 1439, 1340, 1230, 1210, 1170, 1160, 840, 796 $cm^{-1}$.

NMR ($CDCl_3$):

δ 1.46 (3H, d, J=7Hz), 3.60 (3H, s), 3.67 (1H, q, J=7Hz), 6.8—7.4 (4H, m).

Example 54

0.490 g of 1 - (6 - methoxy - 2 - naphthyl) - 2 - (methanesulfonyloxy) - 1 - propanone dimethyl acetal and 0.140 g of calcium carbonate were heated under reflux for 5 hours in 10 ml of a mixture of water and dimethyl formamide (1:4 by weight). Water (20 ml) was added, and the mixture was extracted with 7 ml of methylene chloride four times. The extracts were washed with 10 ml of water four times, dried over anhydrous magnesium sulfate, and concentrated under reduced pressure. The residue was purified by column chromatography (silica gel, methylene chloride) to give 0.323 g of methyl alpha - (6 - methoxy - 2 - naphthyl)propionate as colorless crystals having a melting point of 65 to 68°C. Yield 94%. The product completely agreed with an authentic sample in IR and NMR spectral data.

Example 55

0.354 g of 1 - (6 - methoxy - 2 - naphthyl) - 2 - methanesulfonyloxy - 1 - propanone dimethyl acetal and 0.10 g of calcium carbonate were heated under refux for 28 hours in 8 ml of a mixture of water and methanol (3:7 by weight). Water (20 ml) was added, and the mixture was extracted with 10 ml of methylene chloride three times. The extracts were washed with 20 ml of water, dried over anhydrous magnesium sulfate and concentrated under reduced pressure to give 0.178 g of methyl alpha - (6 - methoxy - 2 - naphthyl)propionate as colorless crystals. Yield 73%.

Example 56

One of two possible diastereomers of 1 - (6 - methoxy - 2 - naphthyl) - 2 - (d - 10 - camphorsulfonyloxy) - 1 - propanone dimethyl acetal was provided (m.p. 102—105°C; $[\alpha]_D^{25}$+32.5° (c=1, chloroform)). 170 mg of this diastereomer and 40 mg of calcium carbonate were heated at a bath temperature of 110°C for 14 hours in 5 ml of a mixture of water and dimethyl formamide (1:4 by weight). Water (20 ml) was added, and the mixture was extracted with 10 ml of diethyl ether four times. The extracts were washed with 10 ml of water four times, dried over anhydrous magnesium sulfate, and concentrated under reduced pressure. The residue was purified by column chromatography (silica gel, methylene chloride) to give 68 mg of methyl(−) - alpha - (6 - methoxy - 2 - naphthyl)propionate as colorless crystals.

Yield: 80.2%.

m.p.: 94.5—95°C.

$[\alpha]_D^{20}$: −78.2° (c=1, chloroform).

IR (KBr):

2970, 1739, 1602, 1450, 1334, 1270, 1231, 1205, 1172, 1158, 1028, 893, 856, 823 $cm^{-1}$.

The product agreed in NMR spectral data with the methyl alpha - (6 - methoxy - 2 - naphthyl)propionate obtained in Example 54.

Example 57

0.31 ml (0.44 g) of ioditrimethylsilane and two drops of cyclohexene were stirred in 5 ml of anhydrous methylene chloride at room temperature in an atmosphere of argon. To the mixture was added dropwise 4 ml of an anhydrous methylene chloride solution of 529 mg of 1 - phenyl - 2 - (p - toluenesulfonyloxy) - 1 - propanone dimethyl acetal. The mixture was stirred at room temperature for 3 hours. Then, 5 ml of a saturated aqueous solution of sodium hydrogen carbonate was added. The organic layer was washed successively with 5 ml of a 10% aqueous solution of sodium thiosulfate, 5 ml of water, 5 ml of a 10% aqueous solution of sodium hydrogen carbonate and 5 ml of water, and dried over anhydrous magnesium sulfate. The product was quantitatively analyzed by gas chromatography (internal standard method) and found to contain 226.4 mg of methyl alpha-phenylpropionate. Yield 91.9%.

Examples 58 to 62

176 mg of 1 - phenyl - 2 - (p - toluenesulfonyloxy) - 1 - propanone dimethyl acetal and each of the agents having affinity for oxygen shown in Table 2 in the amounts indicated were stirred in 4 ml of anhydrous methylene chloride at each of the temperatures shown in Table 2. Water (5 ml) was added, and the mixture was extracted with 5 ml of methylene chloride four times. The extracts were washed with 10 ml of water, and dried over anhydrous magnesium sulfate.

The product was analyzed for methyl alpha-phenylpropionate by gas chromatography in the same way as in Example 57. The results are shown in Table 2.

TABLE 2

| Example | Agent having affinity for oxygen (mole ratio) | Reaction conditions | | Yield of methyl alpha-phenylpropionate (%) |
|---|---|---|---|---|
| | | Temperature (°C) | Time (hours) | |
| 58 | $AlCl_3$ (0.6 | 0 | 1 | 52.5 |
| 59 | $AlCl_3$ (0.7) | Refluxing temperature | 1 | 63.6 |
| 60 | $AlCl_3$ (1.0) | Refluxing temperature | 0.7 | 69.3 |
| 61 | $AlCl_3$ (1.4) | 0 | 1 | 68.0 |
| 62 | $TiCl_4$ (3.0) | Refluxing temperature | 10 | 16.8 |

Example 63

3.849 g of 1 - (4 - chlorophenyl) - 2 - (p - toluenesulfonyloxy) - 1 - propanone dimethyl acetal was stirred at room temperature in an argon atmosphere in 10 ml of anhydrous methylene chloride. A solution obtained by dissolving 1.71 ml (2.40 g of iodotrimethylsilane and 2 drops of cyclohexene in 2 ml of anhydrous methylene chloride was added dropwise at room temperature, and the mixture was stirred for 30 minutes. The same work-up as in Example 57 gave an oily crude product, which was found by its NMR spectrum to contain 1.61 g of methyl alpha - (p - chlorophenyl)propionate. Yield 81.0%.

Example 64

0.20 ml (0.28 g) of iodotrimethylsilane and one drop of cyclohexene were stirred in 8 ml of anhydrous methylene chloride at room temperature in an argon atmosphere. Then, 6 ml of an anhydrous methylene chloride solution of 490 mg (1.00 millimole) of 1 - (6 - methoxy - 2 - naphthyl) - 2 - (d - 10 - camphorsulfonyloxy) - 1 - propanone dimethyl acetal having an $[\alpha]_D^{25}$ of +32.5° (c=1.00, chloroform) was added dropwise, and the mixture was stirred at room temperature for 1 hour. Ten milliliters of a saturated aqueous solution of sodium hydrogen carbonate was added. The same work-up as in Example 57 followed by purification with column chromatography (silica gel, methylene chloride) to give 230 mg of methyl (R) (−) - alpha - (6 - methoxy - 2 - naphthyl)propionate as colorless crystals having a melting point of 85 to 92°C. Yield 94.2%. This product was found to be optically pure by NMR spectroscopy using an optically active shifting agent Eu $(TFC)_3$.

Example 65

By a similar operation to Example 17, 1 - (4 - methoxyphenyl) - 2 - hydroxy - 1 - propanone dimethyl acetal was prepared as a pale yellow oil from 1 - (4 - methoxyphenyl) - 2 - bromo - 1 - propanone. Without purification, the crude product was used in the reaction of Example 66.

Example 66

By a similar operation to Example 7, 1 - (4 - methoxyphenyl) - 2 - methanesulfonyloxy - 1 - propanone dimethyl acetal was prepared as a light yellow oil from the 1 - (4 - methoxyphenyl) - 2 - hydroxy - 1 - propanone dimethyl acetal obtained in Example 65. Yield 80.1% [from 1 - (4 - methoxyphenyl) - 2 - bromo - 1 - propanone].

IR (neat):

2935, 1615, 1517, 1358, 1257, 1176, 1100, 1065, 145, 915 $cm^{-1}$.

NMR ($CDCl_3$):

δ 1.18 (3H, d, J=6Hz), 3.09 (3H, s), 3.21 (3H, s), 3.28 (3H, s), 3.80 (3H, s), 4.97 (1H, q, J=6Hz), 6.87 (2H, d, J=9Hz), 7.37 (2H, d, J=9Hz).

Example 67

1.007 g (3.308 millimoles) of 1 - (4 - methoxyphenyl) - 2 - methanesulfonyloxy - 1 - propanone dimethyl acetal and 330 mg (3.30 millimoles) of calcium carbonate were refluxed with stirring for 20 hours in 10 ml of a mixture of water and methanol (3:7 by weight). By the same work-up and purification as in example 49, 597 mg of methyl alpha - (4 - methoxyphenyl)propionate was obtained as a colorless oil. Yield 92.9%.

NMR ($CDCl_3$):

δ 1.47 (3H, d, J=7Hz), 3.61 (3H, s), 3.67 (1H, q, J=7Hz), 3.73 (3H, s), 6.83 (2H, d, J=9Hz), 7.19 (2H, d, J=9Hz).

Example 68

In a similar way to Example 20, 1 - (2 - thienyl) - 2 - (p - toluenesulfonyloxy) - 1 - propanone dimethyl acetal was prepared as a colorless oil in a yield of 64% from 1 - (2 - thienyl) - 2 - hydroxy - 1 - propanone dimethyl acetal.

IR (neat):

1360, 1190, 1180, 1060, 985, 925, 905, 820, 790, 710, 665, 565 $cm^{-1}$.

NMR ($CDCl_3$):

δ 1.19 (3H, d, J=7Hz), 2.40 (3H, s), 3.08 (3H, s), 3.14 (3H, s), 4.98 (1H, q, J=7Hz), 6.94 (2H, d, J=3Hz), 7.2—7.4 (3H, m), 7.80 (2H, d, J=8Hz).

Example 69

In a similar way to Example 20, 1 - (2 - thienyl) - 2 - (benzenesulfonyloxy) - 1 - propanone dimethyl acetal was prepared as a colorless oil in a yield of 83% from 1 - (2 - thienyl) - 2 - hydroxy - 1 - propanone dimethyl acetal and benzenesulfonyl chloride.

IR (neat):

1360, 1190, 1060, 980, 925, 900, 590, 555 $cm^{-1}$.

NMR ($CDCl_3$):

δ 1.20 (3H, d, J=7Hz), 3.08 (3H, s), 3.14 (3H, s), 5.01 (1H, q, J=7Hz), 6.8—7.1 (2H, m), 7.2—7.4 (1H, m), 7.4—7.7 (3H, m), 7.9—8.1 (2H, m).

Example 70

Metallic sodium (1.30 g, 56.5 millimoles) was dissolved in 30 ml of anhydrous ethanol, and the mixture was stirred at room temperature. To the solution was added dropwise over 15 minutes 20 ml of an anhydrous ethanol solution of 5.33 g (0.025 mole) of alpha-bromopropiophenone, and the mixture was stirred further for 40 minutes at room temperature. Water (100 ml) was added, and the mixture was extracted with 60 ml of diethyl ether twice. The extracts were washed with water, dried over anhydrous magnesium sulfate and anhydrous potassium carbonate, and concentrated under reduced pressure. The residue was dissolved in 30 ml of anhydrous ethanol containing a catalytic amount of metallic sodium dissolved therein, and the solution was stirred for 2 days at room temperature. Usual work-up as in Example 3 gave 4.497 g of 1 - phenyl - 2 - hydroxy - 1 - propanone diethyl acetal as a colorless oil. Yield: 80.2%.

IR (neat):

2960, 1452, 1120, 1092, 1057, 1040, 772, 709 $cm^{-1}$.

NMR ($CDCl_3$):

δ 0.94 (3H, d, J=6Hz), 1.05—1.40 (6H, m), 2.61 (1H, broad s), 3.22—3.83 (4H, m), 4.05 (1H, q, J=6Hz), 7.1—7.7 (5H, m).

Example 71

Acetanilide (3.33 g) and 4.0 ml (5.2 g) of alpha-chloropropionyl chloride were stirred at room

temperature in 20 ml of carbon disulfide. Then, 7.0 g of finely pulverized aluminum chloride was added in portions over 2 minutes. The mixture was stirred at room temperature for 25 minutes, and heated under reflux for 50 minutes with stirring. After cooling, the reaction mixture separated into two layers. The upper layer was removed by decantation, and the lower layer which was black and tarry was poured into ice water. The precipitated crystals were collected by filtration. Recrystallization from ethanol gave 1.860 g of 1 - (4 - acetylaminophenyl) - 2 - chloro - 1 - propanone as colorless crystals. The upper layer of the reaction mixture, the filtrate formed during collection of the crystals, and the mother liquor of recrystallization were combined, and extracted with 50 ml of methylene chloride three times. The extracts were washed with 50 ml of water, dried over anhydrous magnesium sulfate, and concentrated under reduced pressure. Recrystallization of the residue from ethanol gave 1.434 g of 1 - (4 - acetylaminophenyl) - 2 - chloro - 1 - propanone. The mother liquor was concentrated under reduced pressure, and the residue was purified by column chromatography (silica gel, methylene chloride and ether). Recrystallization from methanol gave 0.868 g of the above product further. The total amount of the product was 4.162 g, and the total yield was 75%.

Colorless crystals.

m.p.: 123—125°C (from ethanol).

IR: (KBr):

1690, 1675, 1595, 1540, 1415, 1320, 1360, 1180, 965, 860, 665 $cm^{-1}$.

NMR ($CDCl_3$):

δ 1.70 (3H, d, J=7Hz), 2.18 (3H, s), 5.18 (1H, q, J=7Hz), 7.63 (2H, d, J=9Hz), 7.95 (2H, d, J=9Hz), 7.8—8.1 (1H, broad s).

For $C_{11}H_{12}ClNO_2$:

| | |
|---|---|
| Calculated: | C, 58.54; H, 5.36; Cl, 15.71; N, 6.21%. |
| Found: | C, 58.59; H, 5.40; Cl, 15.63; N, 6.23%. |

Example 72

By a similar operation to Example 17, 1 - (4 - acetylaminophenyl) - 2 - hydroxy - 1 - propanone dimethyl acetal was prepared as a glassy substance from 1 - (4 - acetylaminophenyl) - 2 - chloro - 1 - propanone. Without purification, the crude product was used in the reaction in Example 73.

NMR ($CDCl_3$):

δ 0.95 (3H, d, J=7Hz), 2.14 (3H, s), 2.94 (1H, broad s), 3.21 (3H, s), 3.33 (3H, s), 4.10 (1H, q, J=7Hz), 7.37 (2H, d, J=8Hz), 7.51 (2H, d, J=8Hz), 8.93 (1H, broad s).

Example 73

All of the 1 - (4 - acetylaminophenyl) - 2 - hydroxy - 1 - propanone dimethyl acetal obtained in Example 72 was dissolved in 5 ml of pyridine, and 0.60 ml (0.89 g) of methanesulfonyl chloride was added. The mixture was stirred at room temperature for 5 hours. The reaction mixture was cooled with ice, and 30 ml of water was added. The temperature was returned to room temperature, and the mixture was stirred for 10 minutes, and extract with 20 ml of ethyl acetate four times. The extracts were dried by adding successively adding anhydrous sodium sulfate and anhydrous magnesium sulfate thereto, and concentrated under reduced pressure. The residue was purified by column chromatography (Florisil, methylene chloride and diethyl ether) to give 1.537 g of 1 - (4 - acetylaminophenyl) - 2 - methanesulfonyloxy - 1 - propanone dimethyl acetal as a colorless glass substance. Yield 93% [from 1 - (4 - acetylaminophenyl) - 2 - chloro - 1 - propanone].

IR (KBr):

1675, 1610, 1540, 1350, 1175, 915, 525 $cm^{-1}$.

NMR ($CDCl_3$):

δ 1.14 (3H, d, J=7Hz), 2.52 (3H, s), 3.08 (3H, s), 3.18 (3H, s), 3.24 (3H, s), 4.94 (1H, q, J=7Hz), 7.36 (2H, d, J=9Hz), 7.54 (2H, d, J=9Hz), 8.52 (1H, broad s).

Example 74

In the same way as in Example 17, 1 - (4 - fluorophenyl) - 2 - hydroxy - 1 - propanone dimethyl acetal was prepared as a pale yellow oil from 1 - (4 - fluorophenyl) - 2 - bromo - 1 - propanone. Without purification, this product was used in the reactions of Examples 70 and 76.

IR (neat):

2975, 2937, 1610, 1512, 1230, 1159, 1105, 1053, 982, 839 $cm^{-1}$.

NMR ($CDCl_3$):

δ 0.93 (3H, d, J=6Hz), 2.53 (1H, broad s), 3.23 (3H, s), 3.33 (3H, s), 4.08 (1H, broad q, J=6Hz), 6.83—7.18 (2H, m), 7.32—7.60 (2H, m).

Example 75

By the same operation as in Example 7, 1 - (4 - fluorophenyl) - 2 - methanesulfonyloxy - 1 - propanone dimethyl acetal was prepared as a colorless oil from the 1 - (4 - chlorophenyl) - 2 - hydroxy - 1 - propanone dimethyl acetal obtained in Example 74. Yield 82.3% [from 1 - (4 - fluorophenyl) - 2 - bromo - 1 - propanone].

IR (neat):
2940, 1610, 1512, 1359, 1228, 1180, 1160, 1098, 1067, 1045, 972, 918 $cm^{-1}$.
NMR ($CDCl_3$):
δ 1.15 (3H, d, J=6Hz), 3.06 (3H, s), 3.21 (3H, s), 3.24 (3H, s), 4.96 (1H, q, J=6Hz), 6.88—7.16 (2H, m), 7.32—7.58 (2H, m).

Example 76
In the same way as in Example 20, 1 - (4 - fluorophenyl) - 2 - (p - toluenesulfonyloxy) - 1 - propanone dimethyl acetal was prepared as colorless crystals having a melting point of 96 to 99°C from the 1 - (4 - fluorophenyl) - 2 - hydroxy - 1 - propanone dimethyl acetal obtained in Example 74. Yield 76.2% [from 1 - (4 - fluorophenyl) - 2 - bromo - 1 - propanone].
m.p.: 101°C (from methanol).
IR (KBr):
2948, 1605, 1510, 1360, 1346, 1192, 1181, 1090, 1053, 932, 918, 820, 786, 670, 561 $cm^{-1}$.
NMR ($CDCl_3$):
δ 1.05 (3H, d, J=6Hz), 2.40 (3H, s), 3.08 (3H, s), 3.10 (3H, s), 4.93 (1H, q, J=6Hz), 6.82—7.08 (2H, m), 7.16—7.46 (4H, m), 7.73 (2H, d, J=8Hz).
For $C_{18}H_{21}FO_3S$:

| | |
|---|---|
| Calculated: | C, 58.68; H, 5.75%. |
| Found: | C, 58.98; H, 5.96%. |

Example 77
8.57 g of 1 - (6 - methoxy - 2 - naphthyl) - 1 - propanone (U.S. Patent 2,683,738) was dissolved in 60 ml of anhydrous dioxane and the solution was stirred at room temperature. To the solution was added 13.57 g of pyridinium hydrobromide perbromide, followed by stirring for an additional one hour. 200 ml of a 3% aqueous solution of sodium bisulfite was added to the mixture and, after stirring at room temperature for 3 hours, the precipitated crystals were filtered, washed with water and dried in vacuo over potassium hydroxide to obtain 11.66 g of 1 - (6 - methoxy - 2 - naphthyl) - 2 - bromo - 1 - propanone having a melting point of 73—75°C as pale yellow crystals. Yield, 99%.
m.p.: 68—69°C (from benzene).
NMR ($CDCl_3$):
δ 1.90 (3H, d, J=7Hz), 3.85 (3H, s), 5.36 (1H, q, J=7Hz), 6.9—7.3 (2H, m), 7.5—8.1 (3H, m), 8.38 (1H, broad s).
For $C_{14}H_{13}BrO_2$:

| | |
|---|---|
| Calculated: | C, 57.35; H, 4.47; Br, 27.26%. |
| Found: | C, 57.27; H, 4.66; Br, 27.23%. |

Example 78
In a similar operation to Example 3, 1 - (6 - methoxy - 2 - naphthyl) - 2 - hydroxy - 1 - propanone dimethoxy acetal was prepared as a colorless oil from 1 - (6 - methoxy - 2 - naphthyl) - 2 - bromo - 1 - propanone. Yield, 100%. The product crystallized after it was purified by column chromatography (Florisil, methylene chloride) and allowed to stand at room temperature.
Colorless crystals.
m.p.: 56—59°C.
IR (KBr):
3500, 1637, 1610, 1488, 1276, 1215, 1175, 1118, 1106, 1040, 859 $cm^{-1}$.
NMR ($CDCl_3$):
δ 0.97 (3H, d, J=7Hz), 2.48 (1H, broad s), 3.20 (3H, s), 3.36 (3H, s), 3.82 (3H, s), 4.14 (1H, q, J=7Hz), 7.00—7.24 (2H, m), 7.40—7.98 (4H, m).
For $C_{16}H_{20}O_4$:

| | |
|---|---|
| Calculated: | C, 69.54; H, 7.30%. |
| Found: | C, 69.25; H, 7.28%. |

Example 79
In a similar operation to Example 7, 1 - (6 - methoxy - 2 - naphthyl) - 2 - methanesulfonyloxy - 1 - propanone dimethyl acetal was prepared as a colorless oil from 1 - (6 - methoxy - 2 - naphthyl) - 2 - hydroxy - 1 - propanone dimethyl acetal. Yield, 84%.
IR (neat):
1640, 1615, 1492, 1360, 1280, 1182, 901, 820 $cm^{-1}$.
NMR ($CDCl_3$):
δ 1.19 (3H, d, J=7Hz), 3.09 (3H, s), 3.22 (3H, s), 3.31 (3H, s), 3.87 (3H, s), 5.05 (1H, q, J=7Hz), 7.04—7.24 (2H, m), 7.42—7.94 (4H, m).
For $C_{17}H_{22}O_6S$:

| | |
|---|---|
| Calculated: | C, 57.61; H, 6.26; S, 9.05%. |
| Found: | C, 57.21; H, 6.02; S, 8.85%. |

Example 80

350 mg of sodium metal was dissolved in 40 ml of anhydrous methanol and the solution was stirred at room temperature. 2.93 g of 1 - (6 - methoxy - 2 - naphthyl) - 2 - bromo - 1 - propanone was added to the solution, followed by stirring for 24 hours. 50 ml of water was added to the mixture which was then extracted with methylene chloride (15 ml×4). The extract was dried over anhydrous magnesium sulfate and anhydrous potassium carbonate and concentrated under reduced pressure to obtain 2.845 g of a crude product of 1 - (6 - methoxy - 2 - naphthyl) - 2 - hydroxy - 1 - propanone dimethyl acetal. 1.11 g of the crude product was dissolved in 3 ml of anhydrous pyridine, followed by stirring at room temperature. 993 mg of d - 10 - camphorsulfonyl chloride was added to the solution and the mixture was stirred for 40 minutes. 20 ml of water was added to the mixture which was then extracted with methylene chloride (10 ml×3). The extract was dried by adding successively anhydrous magnesium sulfate and anhydrous potassium carbonate to the extract and concentrated under reduced pressure. The residual oil was purified by column chromatography (Florisil, methylene chloride) to yield 1.467 g of 1 - (6 - methoxy - 2 - naphthyl) - 2 - (d - 10 - camphorsulfonyloxy) - 1 - propanone dimethyl acetal as a colorless glass-like substance. Yield 76.8% [from 1 - (6 - methoxy - 2 - naphthyl) - 2 - bromo - 1 - propanone]. The product was found to be a 1:1 mixture of two diastereomers by high performance liquid chromatography and NMR spectrum.

1.067 g of the product thus obtained was dissolved in 4 ml of methanol and the solution was allowed to stand for 2 days at 2 to 4°C to obtain 355 mg of colorless crystals having a melting point of 90—95°C. The crystals were further recrystallized from methanol to obtain 220 mg of one of the diastereomers as a pure product.

m.p.: 102—105°C.

$[\alpha]_D^{25}$+32.5° (c=1, chloroform).

IR (KBr):

2950, 1753, 1634, 1611, 1487, 1354, 1273, 1219, 1180, 1166, 1068, 1042, 990, 919, 899, 862, 840 $cm^{-1}$.

NMR ($CDCl_3$):

δ 0.87 (3H, s), 1.12 (3H, s), 1.23 (3H, d, J=6Hz), 1.3—2.7 (7H, m), 3.04 (1H, d, J=15Hz), 3.24 (3H, s), 3.35 (3H, s), 3.86 (3H, s), 3.86 (1H, d, J=15Hz), 5.15 (1H, q, J=6Hz), 7.04—7.26 (2H, m), 7.46—7.98 (4H, m).

For $C_{26}H_{33}O_7S$:

| | |
|---|---|
| Calculated: | C, 63.78; H, 6.79; S, 6.55%. |
| Found: | C, 63.66; H, 7.06; S, 6.57%. |

The other diastereomer was separated from the mixture as a colorless oil by high performance liquid chromatography [column: Microporasil manufactured by Waters Co.; column size: 7.8 mm×30 cm; developing solvent: hexane+ethyl acetate (9:1)].

$[\alpha]_D^{25}$+4.2° (c=0.143, chloroform).

IR (KBr):

2950, 1750, 1631, 1608, 1485, 1352, 1271, 1216, 1172, 1062, 1040, 985, 914, 895, 855, 810 $cm^{-1}$.

NMR ($CDCl_3$):

δ 0.91 (3H, s), 1.14 (3H, s), 1.26 (3H, d, J=6Hz), 1.3—2.7 (7H, m), 3.29 (1H, d, J=15Hz), 3.30 (3H, s), 3.42 (3H, s), 3.74 (1H, d, J=15Hz), 3.96 (3H, s), 5.20 (1H, q, J=6Hz), 7.10—7.30 (2H, m), 7.50—8.02 (4H, m).

Example 81

1.922 g (5.000 millimoles) of 1 - (4 - chlorophenyl) - 2 - (p - toluenesulfonyloxy) - 1 - propanone dimethyl acetal and 500 mg (5.00 millimoles) of calcium carbonate were heated with stirring at 110°C (bath temperature) for 3 days in a mixture of dimethyl formamide and water (4:1 by weight). Usual work-up as in Example 36 gave 337 mg of a colorless oil. The oil was found to contain 238 mg of methyl alpha - (4 - chlorophenyl)propionate by NMR spectroscopy. Yield 12.0%.

Example 82

By a similar operation to Example 17, 1 - (4 - chlorophenyl) - 2 - hydroxy - 1 - propanone dimethyl acetal was prepared as a colorless oil from 1 - (4 - chlorophenyl) - 2 - bromo - 1 - propanone. Without further purification, this product was used in the reaction of Example 83.

IR (neat):

3450, 2975, 2930, 1600, 1492, 1398, 1108, 1095, 1052, 1016, 980, 829, 743 $cm^{-1}$.

Example 83

In the same way as in Example 20, 1 - (4 - chlorophenyl) - 2 - (p - toluenesulfonyloxy) - 1 - propanone dimethyl acetal was prepared as white crystals having a melting point of 76 to 77°C from the 1 - (4 - chlorophenyl) - 2 - hydroxy - 1 - propanone dimethyl acetal obtained in Example 82. Yield 86% [from 1 - (4 - chlorophenyl) - 2 - bromo - 1 - propanone].

m.p.: 78—79°C (from methanol).

IR (KBr):

2945, 1601, 1493, 1364, 1347, 1195, 1188, 1094, 1057, 1110, 936, 919, 822, 789, 669, 565 $cm^{-1}$.

NMR ($CDCl_3$):

δ 1.05 (3H, d, J=6Hz), 2.40 (3H, s), 3.08 (3H, s), 3.10 (3H, s), 4.93 (1H, q, J=6Hz), 7.25 (4H, s), 7.27 (2H, d, J=8Hz), 7.76 (2H, d, J=8Hz).

For $C_{18}H_{21}SO_3Cl$:

| | |
|---|---|
| Calculated: | C, 56.17; H, 5.50; Cl, 9.21; S, 8.33%. |
| Found: | C, 56.22; H, 5.48; Cl, 9.13; S, 8.32%. |

Example 84

In the same way as in Example 71, 1 - [4 - (1 - oxo - 2 - isoindolinyl)phenyl] - 2 - chloro - 1 - propanone was prepared as colorless crystals in a yield if 89% from 2 - phenyl - 1 - isoindolinone and alpha-chloropropionyl chloride.

Example 85

In the same way as in Example 17, 1 - [4 - (1 - oxo - 2 - isoindolinyl)phenyl] - 2 - hydroxy - 1 - propanone dimethyl acetal was obtained from 1 - [4 - (1 - oxo - 2 - isoindolinyl)phenyl] - 2 - chloro - 1 - propanone in a yield of 83% as colorless crystals having a melting point of 130 to 135°C.

IR (KBr):

3530, 1680, 1515, 1385, 1310, 1120, 1045, 740 $cm^{-1}$.

NMR ($CDCl_3$):

δ 0.96 (3H, d, J=7Hz), 2.36 (1H, broad s), 3.20 (3H, s), 3.33 (3H, s), 4.10 (1H, broad q, J=7Hz), 4.80 (2H, s), 7.4—7.6 (5H, m), 7.8—8.0 (3H, m).

For $C_{19}H_{21}NO_4$:

| | |
|---|---|
| Calculated: | C, 69.70; H, 6.47; N, 4.28%. |
| Found: | C, 69.63; H, 6.49; N, 4.21%. |

Example 86

In the same way as in Example 73, 1 - [4 - (1 - oxo - 2 - isoindolinyl) - phenyl] - 2 - methanesulfonyloxy - 1 - propanone dimethyl acetal was prepared in a yield of 95% as colorless crystals from 1 - [4 - (1 - oxo - 2 - isoindolinyl)phenyl] - 2 - hydroxy - 1 - propanone dimethyl acetal and methanesulfonyl chloride.

IR (KBr):

1695, 1520, 1390, 1350, 1340, 1175, 975, 910, 740 $cm^{-1}$.

NMR ($CDCl_3$):

δ 1.20 (3H, d, J=7Hz), 3.11 (3H, s), 3.24 (3H, s), 3.30 (3H, s), 4.81 (2H, s), 5.00 (1H, q, J=7Hz), 7.4—7.6 (5H, m), 7.8—8.0 (3H, m).

Example 87

In the same way as in Example 48, methyl alpha - [4 - (1 - oxo - 2 - isoindolinyl)phenyl]propionate was prepared in a yield of 86% as colorless crystals from 1 - [4 - (1 - oxo - 2 - isoindolinyl)phenyl] - 2 - methanesulfonyloxy - 1 - propanone dimethyl acetal.

Example 88

By the same operation as in Example 17, 1 - (4 - biphenylyl) - 2 - hydroxy - 1 - propanone dimethyl acetal was prepared as colorless crystals having a melting point of 78.5 to 80°C in a yield of 95% from 1 - (4 - biphenylyl) - 2 - bromo - 1 - propanone.

m.p.: 78.5—80°C (from ether/n-hexane).

NMR ($CDCl_3$):

δ 1.00 (3H, d, J=7Hz), 2.44 (1H, d, J=3.1Hz), 3.23 (3H, s), 3.37 (3H, s), 4.12 (1H, q, d, J=7Hz, 3.1Hz), 7.2—7.7 and 7.55 (m and s, 9H).

IR (KBr):

1118, 1102, 1045, 1007, 979, 838, 770, 735, 695 $cm^{-1}$.

For $C_{17}H_{20}O_3$:

| | |
|---|---|
| Calculated: | C, 74.97; H, 7.40%. |
| Found: | C, 74.88; H, 7.27%. |

Example 89

In the same way as in Example 7, 1 - (4 - biphenylyl) - 2 - methanesulfonyloxy - 1 - propanone dimethyl acetal was prepared as a colorless oil from 1 - (4 - biphenylyl) - 2 - hydroxy - 1 - propanone dimethyl acetal. Without purification, this product was used in the reaction of Example 90.

IR (neat):

1490, 1357, 1335, 1177, 1123, 1100, 1065, 1045, 971, 916, 847, 811, 773, 753, 740, 702, 538, 527 $cm^{-1}$.

NMR ($CDCl_3$):

δ 1.21 (3H, d, J=7Hz), 3.03 (3H, s), 3.24 (3H, s), 3.31 (3H, s), 5.01 (1H, q, J=7Hz), 7.1—7.7 (m)—7.56 (s) total 9H.

Example 90

In a similar way to Example 21, methyl alpha - (4 - biphenylyl)propionate was prepared as a colorless oil from the 1 - (4 - biphenylyl) - 2 - methanesulfonyloxy - 1 - propanone dimethyl acetal obtained in Example 89. Yield 80.5% [from 1 - (4 - biphenylyl) - 2 - hydroxy - 1 - propanone dimethyl acetal].

NMR ($CDCl_3$):

δ 1.47 (3H, d, J=7Hz), 3.55 (3H, s), 3.68 (1H, q, J=7Hz), 7.1—7.56 (9H, m).

IR (neat):

1741, 1490, 1215, 1167, 765, 701 $cm^{-1}$.

Example 91

By a similar operation to Example 57, methyl alpha - (4 - biphenylyl)propionate was prepared from the 1 - (4 - biphenylyl) - 2 - methanesulfonyloxy - 1 - propanone dimethyl acetal obtained in Example 89. Yield 68% [from 1 - (4 - biphenylyl) - 2 - hydroxy - 1 - propanone dimethyl acetal].

Example 92

By a similar operation as in Example 17, 1,1 - dimethoxy - 2 - hydroxy - 1,2,3,4 - tetrahydronaphthalene was prepared from 2 - bromo - 1 - oxo - 1,2,3,4 - tetrahydronaphthalene. Without purification, this product was used in the reaction shown in Example 93.

IR (neat):

1138, 1080, 1058, 767 $cm^{-1}$.

Example 93

In a similar way to Example 7, 1,1 - dimethoxy - 2 - methanesulfonyloxy - 1,2,3,4 - tetrahydronaphthalene was prepared as colorless crystals having a melting point of 113 to 114.5°C from the 1,1 - dimethoxy - 2 - hydroxy - 1,2,3,4 - tetrahydronaphthalene obtained in Example 92. Yield 46.5% [from 2 - bromo - 1 - oxo - 1,2,3,4 - tetrahydronaphthalene].

m.p.: 113—114.5°C (from methylene chloride/diethyl ether/n-hexane).

IR (KBr):

1361, 1340, 1206, 1175, 1141, 1033, 1060, 1050, 981, 959, 949, 917, 847, 781, 768, 625, 546, 530, 510 $cm^{-1}$.

NMR ($CDCl_3$):

δ 2.37 (2H, m), 2.9 (2H, m), 2.94 (3H, s), 2.98 (3H, s), 3.43 (3H, s), 5.27 (1H, t, J=3Hz), 7.17 (3H, m), 7.63 (1H, m).

For $C_{13}H_{18}O_5S$:

| | |
|---|---|
| Calculated: | C, 54.53; H, 6.34; S, 11.20%. |
| Found: | C, 54.73; H, 6.36; S, 11.10%. |

Example 94

By a similar operation to Example 54, methyl indane-1-carboxylate was prepared from 1,1 - dimethoxy - 2 - methanesulfonyloxy - 1,2,3,4 - tetrahydronaphthalene. Yield 4%.

Example 95

In a similar operation to Example 58, methyl alpha-phenylpropionate was prepared in 51.4% yield from 1 - phenyl - 2 - (p - toluenesulfonyloxy) - 1 - propanone dimethyl acetal and tin chloride.

Example 96

In a similar way to Example 77, 2 - bromo - 1 - (4 - difluoromethoxyphenyl) - 1 - propanone was prepared as a colorless oil from 1 - (4 - difluoromethoxyphenyl) - 1 - propanone.

NMR ($CDCl_3$):

δ 1.92 (3H, d, J=7Hz), 5.23 (1H, q, J=7Hz), 6.58 (1H, t, J=72Hz), 7.15 (2H, d, J=9Hz), 8.00 (2H, d, J=9Hz).

Example 97

By a similar operation to Example 17, 1 - (4 - difluoromethoxyphenyl) - 2 - hydroxy - 1 - propanone dimethyl acetal was prepared as a colorless oil from 2 - bromo - 1 - (4 - difluoromethoxyphenyl) - propanone.

IR (neat):

3600—3200, 1515, 1385, 1230, 1130, 1050, 840 $cm^{-1}$.

NMR ($CDCl_3$):

δ 0.95 (3H, d, J=7Hz), 2.37 (1H, d, J=3Hz), 3.23 (3H, s), 3.33 (3H, s), 4.10 (1H, dq, J=3 and 7Hz), 6.52 (1H, t, J=74Hz), 7.10 (2H, d, J=9Hz), 7.49 (2H, d, J=9Hz).

Example 98

In a similar way to Example 7, 1 - (4 - difluoromethoxyphenyl) - 2 - methanesulfonyloxy - 1 - propanone dimethyl acetal was prepared as a colorless oil from the 1 - (4 - difluoromethoxyphenyl) - 2 -

hydroxy - 1 - propanone dimethyl acetal obtained in Example 97. Yield 55% [from 1 - (4 - difluoromethoxyphenyl) - 1 - propanone].

IR (neat):

1515, 1360, 1235, 1180, 1130, 1100, 1070, 1045, 975, 920, 540, 530 $cm^{-1}$.

NMR ($CDCl_3$):

δ 1.18 (3H, d, J=7Hz), 3.09 (3H, s), 3.15 (3H, s), 3.18 (3H, s), 4.98 (1H, q, J=7Hz), 6.53 (1H, t, J=74Hz), 7.10 (2H, d, J=9Hz), 7.45 (2H, d, J=9Hz).

Example 99

In a similar way to Example 77, 2 - bromo - 1 - (4 - difluoromethoxyphenyl) - 3 - methyl - 1 - butanone was prepared as a colorless oil from 1 - (4 - difluoromethoxyphenyl) - 3 - methyl - 1 - butanone.

IR (neat):

1690, 1605, 1230, 1120, 1055 $cm^{-1}$.

NMR ($CDCl_3$):

δ 1.02 (3H, d, J=7Hz), 1.21 (3H, d, J=7Hz), 2.1—2.8 (1H, m), 4.85 (1H, d, J=9Hz), 6.60 (1H, t, J=74Hz), 7.18 (2H, d, J=9Hz), 8.03 (2H, d, J=9Hz).

Example 100

By a similar operation to Example 17, 1 - (4 - difluoromethoxyphenyl) - 2 - hydroxy - 3 - methyl - 1 - butanone dimethyl acetal was prepared as a colorless oil from 2 - bromo - 1 - (4 - difluoromethoxyphenyl) - 3 - methyl - 1 - butanone.

IR (neat):

3600—3300, 1515, 1390, 1230, 1130, 1050 $cm^{-1}$.

NMR ($CDCl_3$):

δ 0.69 (3H, d, J=6Hz), 0.88 (3H, d, J=6Hz), 1.2—1.7 (1H, m), 2.50 (1H, d, J=3Hz), 3.22 (3H, s), 3.24 (3H, s), 3.70 (1H, dq, J=3 and 6Hz), 6.50 (1H, t, J=74Hz), 7.07 (2H, d, J=9Hz), 7.52 (2H, d, J=9Hz).

Example 101

In a similar way to Example 13, 1 - (4 - difluoromethoxyphenyl) - 2 - methanesulfonyloxy - 3 - methyl - 1 - butanone dimethyl acetal was prepared as a colorless oil from 1 - (4 - difluoromethoxyphenyl) - 2 - hydroxy - 3 - methyl - 1 - butanone dimethyl acetal.

Yield 76% [from 1 - (4 - difluoromethoxyphenyl) - 3 - methyl - 1 - butanone].

On standing at low temperatures, this product crystallized.

m.p.: 60—61°C.

IR (KBr):

1515, 1350, 1230, 1180, 1145, 1080, 1055, 1035, 975 $cm^{-1}$.

NMR ($CDCl_3$):

δ 0.67 (3H, d, J=6Hz), 0.91 (3H, d, J=6Hz), 1.4—1.9 (1H, m), 3.17 (3H, s), 3.19 (3H, s), 3.22 (3H, s), 4.75 (1H, d, J=4Hz), 6.50 (1H, t, J=74Hz), 7.08 (2H, d, J=9Hz), 7.50 (2H, d, J=9Hz).

Example 102

By a similar operation to Example 54, methyl alpha - (4 - difluoromethoxyphenyl)propionate was prepared in a yield of 51% from the 1 - (4 - difluoromethoxyphenyl) - 2 - methanesulfonyloxy - 1 - propanone dimethyl acetal obtained in Example 98.

IR (neat):

1745, 1515, 1385, 1230, 1160, 1130, 1050 $cm^{-1}$.

NMR ($CDCl_3$):

δ 1.46 (3H, d, J=7Hz), 3.61 (3H, s), 3.67 (1H, q, J=7Hz), 6.43 (1H, t, J=74Hz), 7.02 (2H, J=9Hz), 7.26 (2H, J=9Hz).

Example 103

0.368 g of 1 - (4 - difluoromethoxyphenyl) - 2 - methanesulfonyloxy - 3 - methyl - 1 - butanone dimethyl acetal and 0.100 g of calcium carbonate were heated under reflux in 3 ml of a mixed solvent of water and methanol (3:7 by weight) for 18 days. Usual work-up followed by chromatographic separation gave 0.132 g of methyl α - (4 - difluoromethoxyphenyl)isovalerate as a colorless oil. Yield 51%.

NMR ($CDCl_3$):

δ 0.71 (3H, d, J=7Hz), 1.02 (3H, d, J=7Hz), 2.0—2.5 (1H, m), 3.14 (1H, d, J=11Hz), 3.63 (3H, s), 6.47 (1H, t, J=74Hz), 7.02 (2H, d, J=9Hz), 7.31 (2H, d, J=9Hz).

Example 104

In 3 ml of a mixed solvent of water and DMF (1:4 by weight), 0.368 g of 1 - (4 - difluoromethoxyphenyl) - 2 - methanesulfonyloxy - 3 - methyl - 1 - butanone and 0.100 g of calcium carbonate were heated at 110°C for 12 days, then refluxed for 3 days. After usual work-up and separation, 78 mg of methyl α - (4 - difluoromethoxyphenyl)isovalerate was obtained. Yield 30%.

Example 105

230 mg of sodium metal was dissolved in 3 ml of anhydrous methanol. To the solution was added an anhydrous methanol solution (2 ml) of 1.43 g of 2 - bromo - 1 - (4 - ethoxyphenyl) - 3 - methyl - 1 - butanone. The mixture was stirred at room temperature for 1.5 hours, then at 50°C for 45 minutes. Usual work-up gave 1.344 g of a crude product of 1 - (4 - ethoxyphenyl) - 2 - hydroxy - 3 - methyl - 1 - butanone dimethyl acetal as a colorless without further purification, oily substance. The crude product was subjected to the reaction in Example 106.

IR (neat):

3600—3300, 1610, 1515, 1485, 1400, 1250, 1175, 1115, 1050, 990, 925, 840, 810 $cm^{-1}$.

NMR ($CDCl_3$):

δ 0.68 (3H, t, J=6Hz), 0.87 (3H, d, J=6Hz), 1.39 (3H, t, J=7Hz), 1.2—1.7 (1H, m), 2.50 (1H, d, J=3Hz), 3.22 (3H, s), 3.24 (3H, s), 3.68 (1H, dd, J=3 and 5Hz), 4.01 (2H, q, J=7Hz), 6.82 (2H, d, J=9Hz), 7.37 (2H, d, J=9Hz).

Example 106

In a similar way as in Example 13, 1 - (4 - ethoxyphenyl) - 2 - methanesulfonyloxy - 3 - methyl - 1 - butanone dimethyl acetal was prepared as colorless crystals from the 1 - (4 - ethoxyphenyl) - 2 - hydroxy - 3 - methyl - 1 - butanone dimethyl acetal obtained in Example 105. Yield 62% [from 2 - bromo - 1 - (4 - ethoxyphenyl) - 3 - methyl - 1 - butanone].

m.p.: 83—87°C.

IR (KBr):

1610, 1335, 1260, 1245, 1175, 955, 850, 840 $cm^{-1}$.

NMR ($CDCl_3$):

δ 0.71 (3H, d, J=6Hz), 0.94 (3H, d, J=6Hz), 1.40 (3H, t, J=7Hz), 1.5—1.9 (1H, m), 3.18 (3H, s), 3.20 (3H, s), 3.24 (3H, s), 4.02 (2H, q, J=7Hz), 4.57 (1H, d, J=3Hz), 6.85 (2H, d, J=9Hz), 7.37 (2H, d, J=9Hz).

Example 107

In 7 ml of a mixture of water and methanol (3:7 by weight), 724 mg of 1 - (4 - ethoxyphenyl) - 2 - methanesulfonyloxy - 3 - methyl - 1 - butanone dimethyl acetal and 200 mg of calcium carbonate were heated under reflux for 11 hours. After usual work-up and purification, 467 mg of methyl α - (4 - ethoxyphenyl)isovalerate was obtained as a colorless oil. Yield 99%.

b.p.: 160°C (bath temperature)/17 Torr.

NMR ($CDCl_3$):

δ 0.70 (3H, d, J=6Hz), 1.01 (3H, d, J=6Hz), 1.38 (3H, t, J=7Hz), 2.0—2.6 (1H, m), 3.07 (1H, d, J=10Hz), 3.30 (3H, s), 3.97 (2H, q, J=7Hz), 6.80 (2H, d, J=9Hz), 7.20 (2H, d, J=9Hz).

For $C_{14}H_{20}O_3$:

| | |
|---|---|
| Calculated: | C, 71.16; H, 8.53%. |
| Found: | C, 70.93; H, 8.52%. |

Example 108

In a similar way as in Example 105, 2.58 g of the crude product of 1 - (4 - methoxyphenyl) - 2 - hydroxy - 3 - methyl - 1 - butanone dimethyl acetal was prepared as a colorless oil from 2.71 g of 2 - bromo - 1 - (4 - methoxyphenyl) - 3 - methyl - 1 - butanone and subjected to the reaction in Example 109 without purification.

IR (neat):

3600—3300, 1615, 1515, 1255, 1175, 1115, 1045, 840 $cm^{-1}$.

NMR ($CDCl_3$):

δ 0.70 (3H, d, J=6Hz), 0.87 (3H, d, J=6Hz), 1.2—1.7 (1H, m), 2.53 (1H, broad s), 3.33 (3H, s), 3.34 (3H, s), 3.69 (1H, d, J=5Hz), 3.78 (3H, s), 6.83 (2H, d, J=9Hz), 7.40 (2H, d, J=9Hz).

Example 109

2.58 g of the 1 - (4 - methoxyphenyl) - 2 - hydroxy - 3 - methyl - 1 - butanone dimethyl acetal obtained in Example 108 was dissolved in 5 ml of anhydrous pyridine, and the solution was stirred under ice-cooling. To the solution was added 2.00 g of methanesulfonic anhydride, and the mixture was stirred for 4 hours under ice-cooling, then for 2 hours at room temperature. To the mixture was added 20 ml of water, and the mixture was stirred for 1 hour at room temperature, extracted with 20 ml of diethyl ether three times. The extracts were washed with 5 ml of water twice and dried over anhydrous magnesium sulfate, and concentrated under reduced pressure. The oily residue was purified by column chromatography (Florisil, methylene chloride) to yield 3.10 g of 1 - (4 - methoxyphenyl) - 2 - methanesulfonyloxy - 3 - methyl - 1 - butanone dimethyl acetal as a colorless oil (upon standing at room temperature, this compound crystallized). Yield 93% [from 2 - bromo - 1 - (4 - methoxyphenyl) - 3 - methyl - 1 - butanone].

m.p.: 71.5—73°C.

IR (KBr):

1620, 1525, 1355, 1260, 1180, 1105, 1055, 980, 970, 960, 945, 865, 850 $cm^{-1}$.

NMR ($CDCl_3$):
δ 0.69 (3H, d, J=6Hz), 0.89 (3H, d, J=6Hz), 1.5—1.9 (1H, m), 3.14 (3H, s), 3.16 (3H, s), 3.21 (3H, s), 3.76 (3H, s), 4.72 (1H, d, J=4Hz), 6.82 (2H, d, J=9Hz), 7.36 (2H, d, J=9Hz).

Example 110

In a similar way as in Example 107, methyl α - (4 - methoxyphenyl)isovalerate was prepared as a colorless oil from 1 - (4 - methoxyphenyl) - 2 - methanesulfonyloxy - 3 - methyl - 1 - butanone dimethyl acetal. Yield 94%.

IR (neat):
1740, 1515, 1255, 1160, 1040, 830 $cm^{-1}$.

NMR ($CDCl_3$):
δ 0.70 (3H, d, J=6Hz), 1.00 (3H, d, J=6Hz), 2.0—2.6 (1H, m), 3.08 (1H, d, J=10Hz), 3.63 (3H, s), 3.78 (3H, s), 6.80 (2H, d, J=9Hz), 7.21 (2H, d, J=9Hz).

Example 111

To a stirred suspension of 0.65 g of sodium hydride (55% in mineral oil) in anhydrous tetrahydrofuran (10 ml) was added 2.7 ml of ethylene glycol under ice-cooling. After stirring for 40 minutes at room temperature, 2.71 g of 2 - bromo - 1 - (4 - methoxyphenyl) - 3 - methyl - 1 - butanone was added to the reaction mixture and the mixture was stirred for 12 hours at 60°C. After addition of water (50 ml), the reaction mixture was extracted with 30 ml of methylene chloride three times. The extracts were washed with 10 ml of water, dried over magnesium sulfate, and concentrated in vacuo. The oil residue was subjected to column chromatography (Florisil, methylene chloride) to yield 719 mg of 2 - hydroxy - 1 - (4 - methoxyphenyl) - 3 - methyl - 1 - butanone ethylene acetal as a colorless oil. Yield 29%.

IR (neat):
3600—3400, 1620, 1520, 1255, 1175, 1040, 845 $cm^{-1}$.

NMR ($CDCl_3$):
δ 0.88 (3H, d, J=6Hz), 0.90 (3H, d, J=6Hz), 1.2—1.7 (1H, m), 2.55 (1H, broad d, J=5Hz), 3.5—4.2 (5H, m), 3.77 (3H, s), 6.83 (2H, d, J=9Hz), 7.36 (2H, d, J=9Hz).

Example 112

In a similar way as in Example 13, 1 - (4 - methoxyphenyl) - 2 - methanesulfonyloxy - 3 - methyl - 1 - butanone ethylene acetal was prepared as a colorless crystals from 2 - hydroxy - 1 - (4 - methoxyphenyl) - 3 - methyl - 1 - butanone ethylene acetal. Yield 78%.

m.p.: 82—83°C.

IR (KBr):
1610, 1510, 1335, 1250, 1170, 1050, 970, 940, 930, 815 $cm^{-1}$.

NMR ($CDCl_3$):
δ 0.91 (6H, d, J=7Hz), 1.5—1.9 (1H, m), 2.99 (3H, s), 3.6—4.2 (4H, m), 3.75 (3H, s), 4.67 (1H, d, J=4Hz), 6.83 (2H, d, J=9Hz), 7.34 (2H, d, J=9Hz).

Example 113

In 5 ml of a mixture of water and methanol (3:7 by weight), 495 mg of 1 - (4 - methoxyphenyl) - 2 - methanesulfonyloxy - 3 - methyl - 1 - butanone ethylene acetal and 150 mg of calcium carbonate were heated under reflux for 148 hours. After usual work-up followed by column-chromatographic purification, 307 mg of 2-hydroxyethyl α - (4 - methoxyphenyl)isovalerate was obtained as a colorless oil. Yield 81%.

IR (neat):
3600—3200, 1735, 1610, 1510, 1255, 1170, 1160, 1035, 830 $cm^{-1}$.

NMR ($CDCl_3$):
δ 0.70 (3H, d, J=7Hz), 1.03 (3H, d, J=7Hz), 2.0—2.5 (1H, m), 2.27 (1H, broad s), 3.13 (1H, d, J=10Hz), 3.5—3.8 (2H, m), 3.76 (3H, s), 4.0—4.3 (2H, m), 6.81 (2H, d, J=9Hz), 7.21 (2H, d, J=9Hz).

Example 114

350 mg of 1 - phenyl - 2 - (p - toluenesulfonyloxy) - 1 - propanone dimethyl acetal and 0.20 ml of trimethylsilyl trifluoromethanesulfonate were stirred in 1 ml of orthoformic acid trimethyl ester at 65°C for 9 hours. After usual work-up, methyl alpha-phenylpropionate was found to be 50.5% yield by GLC analysis in a same way as in Example 57.

Example 115

In a similar operation as in Example 58, methyl alpha-phenylpropionate was prepared in 80% yield from 1 - phenyl - 2 - (p - toluenesulfonyloxy) - 1 - propanone dimethyl acetal and ferric chloride.

**Claims**

1. A process for preparing an alpha-aromatic group substituted alkanoic acid or ester of the general formula

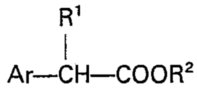

(I)

wherein Ar represents an aromatic group and $R^1$ represents a hydrogen atom or a saturated aliphatic group, or Ar and $R^1$ may form a condensed ring together with the carbon atom to which they are bonded; and $R^2$ represents a hydrogen atom, an alkyl group, or a hydroxyalkyl group, characterized in that an alpha-sulfonyloxyketone acetal of the general formula

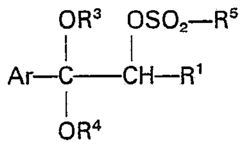

(II)

wherein $R^3$ and $R^4$ independently represent alkyl groups or together represent an alkylene group; $R^5$ represents a substituted or unsubstituted alkyl group or an aromatic group; and Ar and $R^1$ are as defined above, is hydrolyzed, or treated with an agent having affinity for oxygen.

2. A process according to claim 1 wherein Ar is a 6-methoxy-2-naphthyl group and $R^1$ is a methyl group.

3. A process according to claim 1 wherein Ar is a 4-lower alkoxyphenyl group and $R^1$ is an isopropyl group.

4. A process according to claim 1 wherein Ar is a 4-lower alkoxyphenyl group and $R^1$ is a methyl group.

5. A compound of the general formula

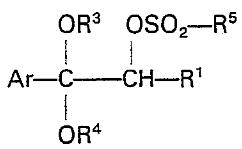

(II)

wherein Ar represents an aromatic group and $R^1$ represents a hydrogen atom or a saturated aliphatic group, or Ar and $R^1$ may form a condensed ring together with the carbon atom to which they are bonded; $R^3$ and $R^4$ independently represent alkyl groups or together represent an alkylene group; and $R^5$ represents a substituted or unsubstituted alkyl group or an aromatic group.

6. A compound of the general formula

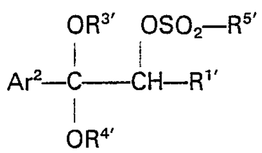

(II-1)

wherein $Ar^2$ represents the group $R^6$—$Ar^1$— or a thienyl group, $Ar^1$ represents a phenylene or naphthylene group; $R^{1'}$ represents a hydrogen atom or a $C_1$—$C_6$ alkyl group; $R^{3'}$ and $R^{4'}$ independently represent $C_1$—$C_6$ alkyl groups or together represent a $C_1$—$C_6$ alkylene group; $R^{5'}$ represents a $C_1$—$C_6$ alkyl group, a haloalkyl group, a d- or l-10-camphoryl group, or a group of the formula

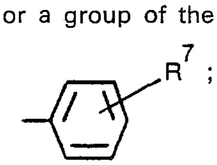

;

$R^6$ represents a hydrogen atom, a halogen atom, a $C_1$—$C_6$ alkyl group, a $C_1$—$C_6$ alkoxy group, a $C_1$—$C_6$ haloalkoxy group, a $C_1$—$C_6$ alkanoylamino group, an oxo-isoindolinyl group, or a phenyl group; and $R^7$ represents a hydrogen atom, a halogen atom, a nitro group or a $C_1$—$C_6$ group.

7. A compound according to claim 6 wherein $Ar^2$ represents a 6-methoxy-2-naphthyl group.

8. A process for producing a compound of formula (II) as defined in claim 5, which comprises (a) reacting a compound of the formula

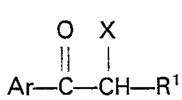

(V)

wherein X represents a halogen atom, Ar and $R^1$ are as defined in claim 5, with an alkali metal alkoxide of the formula

$R^3OM$

wherein M represents an alkali metal and $R^3$ is as defined in claim 5, in the presence of an alcohol of the formula

$R^3OH$

wherein $R^3$ is as defined above, or reacting the compound of formula (V) with an alkali metal alkoxide of the formula

$R^3OM$

wherein $R^3$ and M are as defined above, to form an epoxy compound of the formula

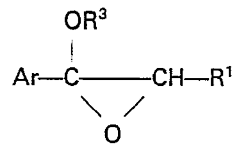 (VII)

wherein Ar, $R^1$ and $R^3$ are as defined above, and then reacting the epoxy compound with an alcohol of the formula

$R^4OH$

wherein $R^4$ is as defined in claim 5, in the presence of an alkali metal alkoxide of the formula

$R^4OM$

wherein $R^4$ is as defined above and M represents an alkali metal, or reacting the compound of formula (V) with an alkylene glycol, and

(b) reacting the resulting compound of the formula

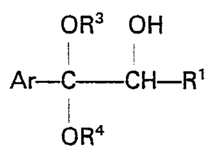 (VI)

wherein Ar, $R^1$, $R^3$ and $R^4$ are as defined above, with a compound of the formula

$R^5—SO_2—Hal$ or $(R^5—SO_2)_2O$

wherein $R^5$ is as defined in claim 5 and Hal represents a halogen atom.

9. A compound according to claim 6 which is 1 - (6 - methoxy - 2 - naphthyl) - 2 - methanesulfonyloxy - 1 - propanone dimethyl acetal.

10. A compound according to claim 6 which is 1 - (6 - methoxy - 2 - naphthyl) - 2 - (d - 10 - camphorsulfonyloxy) - 1 - propanone dimethyl acetal.

11. A compound according to claim 6 which is 1 - (6 - methoxy - 2 - naphthyl) - 2 - (d - 10 - camphorsulfonyloxy) - 1 - propanone dimethyl acetal having an optical rotation $[\alpha]_D^{25}$ of +32.5 degrees (C=1, chloroform).

12. A compound according to claim 6 which is 1 - (6 - methoxy - 2 - naphthyl) - 2 - (d - 10 - camphorsulfonyloxy) - 1 - propanone dimethyl acetal having an optical rotation $[\alpha]_D^{25}$ of +4.2 degrees (C=0.143, chloroform).

**Patentansprüche**

1. Verfahren zur Herstellung einer mit einer α-aromatischen Gruppe substituierten Alkansäure oder eines Esters derselben mit der allgemeinen Formel

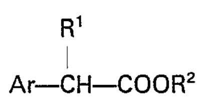 (I)

worin Ar eine aromatische Gruppe darstellt, $R^1$ ein Wasserstoffatom oder eine gesättigte aliphatische Gruppe bedeutet oder Ar und $R^1$ zusammen mit dem Kohlenstoffatom, an das sie gebunden sind, einen

kondensierten Ring bilden können; und $R^2$ ein Wasserstoffatom, eine Alkylgruppe oder eine Hydroxyalkylgruppe ist, dadurch gekennzeichnet, daß an α-Sulfonyloxyketonacetal der allgemeinen Formel

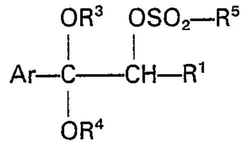
(II)

worin $R^3$ und $R^4$ unabhängig voneinander Alkylgruppen oder zusammen eine Alkylengruppe darstellen, $R^5$ eine substituierte oder unsubstituierte Alkylgruppe oder eine aromatische Gruppe bedeutet, und Ar und $R^1$ wie oben definiert sind, hydrolysiert oder mit einem Mittel behandelt wird, das eine Affinität für Sauerstoff besitzt.

2. Verfahren nach Anspruch 1, dadurch gekennzeichnet, daß Ar eine 6-Methoxy-2-naphthylgruppe und $R^1$ eine Methylgruppe ist.

3. Verfahren nach Anspruch 1, dadurch gekennzeichnet, daß Ar eine 4-Niederalkoxyphenylgruppe und $R^1$ eine Isopropylgruppe ist.

4. Verfahren nach Anspruch 1, dadurch gekennzeichnet, daß Ar eine 4-Niederalkoxyphenylgruppe und $R^1$ eine Methylgruppe ist.

5. Verbindung der allgemeinen Formel

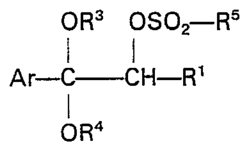
(II)

worin Ar eine aromatische Gruppe und $R^1$ ein Wasserstoffatom oder eine gesättigte aliphatische Gruppe darstellt oder Ar und $R^1$ zusammen mit dem Kohlenstoffatom, an das sie gebunden sind, einen kondensierten Ring bilden können; $R^3$ und $R^4$ unabhängig voneinander für Alkylgruppen stehen oder zusammen eine Alkylengruppe darstellen; und $R^5$ eine substituierte oder unsubstituierte Alkylgruppe oder eine aromatische Gruppe ist.

6. Verbindung der allgemeinen Formel

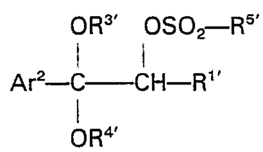
(II-1)

worin $Ar^2$ die Gruppe $R^6$—$Ar^1$— oder eine Thienylgruppe darstellt, $Ar^1$ eine Phenylen- oder Naphthylengruppe bedeutet; $R^{1'}$ ein Wasserstoffatom oder eine $C_1$—$C_6$ Alkylgruppe ist; $R^{3'}$ und $R^{4'}$ unabhängig voneinander $C_1$—$C_6$ Alkylgruppen sind oder zusammen für eine $C_1$—$C_6$ Alkylengruppe stehen; $R^{5'}$ eine $C_1$—$C_6$ Alkylgruppe, eine Haloalkylgruppe, eine d- oder l-10-Camphorylgruppe oder eine Gruppe der Formel

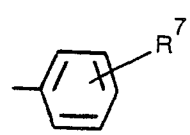
;

ist; $R^6$ ein Wasserstoffatom, ein halogenatom, eine $C_1$—$C_6$ Alkylgruppe, eine $C_1$—$C_6$-Alkoxygruppe, eine $C_1$—$C_6$-Haloalkoxygruppe, eine $C_1$—$C_6$-Alkanoylaminogruppe, eine Oxoisoindolinylgruppe oder eine Phenylgruppe bedeutet, und $R^7$ für ein Wasserstoffatom, ein Halogenatom, eine Nitrogruppe oder eine $C_1$—$C_6$-Alkylgruppe steht.

7. Verbindung nach Anspruch 6, worin $Ar^2$ eine 6-Methoxy-2-naphthylgruppe ist.

8. Verfahren zur Herstellung einer Verbindung der Formel (II), wie in Anspruch 5 definiert, welches umfaßt

a) die Reaktion einer Verbindung der Formel

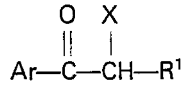
(V)

worin X ein Halogenatom ist und Ar und $R^1$ wie in Anspruch 5 definiert sind, mit einem Alkalimetallalkoxid der Formel

$R^3OM$

worin M für ein Alkalimetall steht und $R^3$ wie in Anspruch 5 definiert ist, in Anwesenheit eines Alkohols der Formel

$R^3OH$

worin $R^3$ wie oben definiert ist, oder die Reaktion einer Verbindung der Formel (V) mit einem Alkalimetallalkoxid der Formel

$R^3OM$

worin $R^3$ und M wie oben definiert sind, unter Bildung einer Epoxyverbindung der Formel

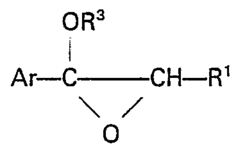 (VII)

worin Ar, $R^1$ und $R^3$ wie oben definiert sind, und die anschließende Reaktion der Epoxyverbindung mit einem Alkohol der Formel

$R^4OH$

worin $R^4$ wie in Anspruch 5 definiert ist, in Anwesenheit eines Alkalimetallalkoxids der Formel

$R^4OM$

worin $R^4$ wie oben definiert ist und M für ein Alkalimetall steht, oder die Reaktion einer Verbindung der Formel (V) mit einem Alkylenglykol und

(b) die Reaktion der resultierenden Verbindung der Formel

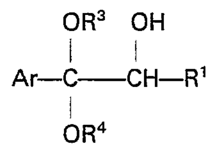 (VI)

worin Ar, $R^1$, $R^3$ und $R^4$ wie oben definiert sind, mit einer Verbindung der Formel

$R^5—SO_2—Hal$ oder $(R^5—SO_2)_2O$

worin $R^5$ wie in Anspruch 5 definiert ist und Hal für ein Halogenatom steht.

9. Verbindung nach Anspruch 6, nämlich 1 - (6 - Methoxy - 2 - naphthyl) - 2 - methansulfonyloxy - 1 - propanondimethylacetal.

10. Verbindung nach Anspruch 6, nämlich 1 - (6 - Methoxy - 2 - naphthyl) - 2 - (d - 10 - camphersulfonyloxy) - 1 - propanondimethylacetal.

11. Verbindung nach Anspruch 6, nämlich 1 - (6 - Methoxy - 2 - naphthyl) - 2 - (d - 10 - camphersulfonyloxy) - 1 - propanondimethylacetal mit einer optischen Drehung $[\alpha]_D^{25}$ von +32,5° (c=1, Chloroform).

12. Verbindung nach Anspruch 6, nämlich 1 - (6 - Methoxy - 2 - naphthyl) - 2 - (d - 10 - camphersulfonyloxy) - 1 - propanondimethylacetal mit einer optischen Drehung $[\alpha]_D^{25}$ von 4,2° (c=0,143, Chloroform).

**Revendications**

1. Procédé de préparation d'un acide alcanoïque substitué par un groupe aromatique un alpha ou d'un ester de cet acide, de formule générale

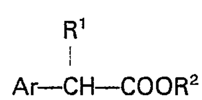 (I)

dans laquelle Ar représente un groupe aromatique et $R^1$ représente un atome d'hydrogène ou un groupe

aliphatique saturé, ou bien Ar et $R^1$ peuvent former un noyau condensé conjointement avec l'atome de carbone auquel ils sont liés; et $R^2$ représente un atome d'hydrogène, un groupe alkyle ou un groupe hydroxyalkyle, caractérisé en ce qu'un acétal d'alpha-sulfonyloxycétone de formule générale

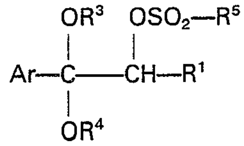

(II)

dans laquelle $R^3$ et $R^4$ représentent, indépendamment, des groupes alkyle ou représentent ensemble un groupe alkylène; $R^5$ représente un groupe alkyle substitué ou non substitué ou un groupe aromatique; et Ar et $R^1$ sont tels que définis ci-dessus, est hydrolysé ou traité avec un agent ayant de l'affinité pour l'oxygène.

2. Procédé suivant la revendication 1, dans lequel Ar est un groupe 6-méthoxy-2-naphtyle et $R^1$ est un groupe méthyle.

3. Procédé suivant la revendication 1, dans lequel Ar est un groupe 4-(alkoxy inférieur)phényle et $R^1$ est un groupe isopropyle.

4. Procédé suivant la revendication 1, dans lequel Ar est un groupe 4-(alkoxy inférieur)phényle et $R^1$ est un groupe méthyle.

5. Composé de formule générale

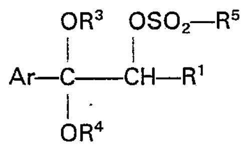

(II)

dans laquelle Ar représente un groupe aromatique et $R^1$ représente un atome d'hydrogène ou un groupe aliphatique saturé, ou bien Ar et $R^1$ peuvent former un noyau condensé conjointement avec l'atome de carbone auquel ils sont liés; $R^3$ et $R^4$ représentent, indépendamment, des groupes alkyle ou répresentent ensemble un groupe alkylène; et $R^5$ représente un groupe alkyle substitué ou non substitué ou un groupe aromatique.

6. Composé de formule générale

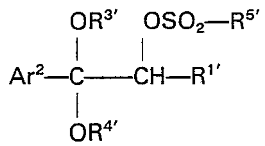

(II-1)

dans laquelle $Ar^2$ représente le groupe $R^6$—$Ar^1$— ou un groupe thiényle, $Ar^1$ représente un groupe phénylène ou naphtylène; $R^{1'}$ représente un atome d'hydrogène ou un groupe alkyle en $C_1$ à $C_6$; $R^{3'}$ et $R^{4'}$ représentent, indépendamment, des groupes alkyle en $C_1$ à $C_6$ ou représentent ensemble un groupe alkylène en $C_1$ à $C_6$; $R^{5'}$ représente un groupe alkyle en $C_1$ à $C_6$, un groupe halogénalkyle, un groupe d- ou l-10-camphoryle ou un groupe de formule

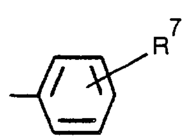

;

$R^6$ représente un atome d'hydrogène, un atome d'halogène, un groupe alkyle en $C_1$ à $C_6$, un groupe alkoxy en $C_1$ à $C_6$, un groupe halogénalkoxy en $C_1$ à $C_6$, un groupe alcanoylamino en $C_1$ à $C_6$, un groupe oxo-isoindolinyle ou un groupe phényle; et $R^7$ représente un atome d'hydrogène, un atome d'halogène, un groupe nitro ou un groupe alkyle en $C_1$ à $C_6$.

7. Composé suivant la revendication 6, dans lequel $Ar^2$ représente un groupe 6-méthoxy-2-naphtyle.

8. Procédé de production d'un composé de formule (II) suivant la revendication 5, qui consiste (a) à faire réagir un composé de formule

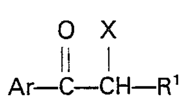

(V)

dans laquelle X représente un atome d'halogène, Ar et $R^1$ sont tels que définis dans la revendication 5, avec un alcoolate de métal alcalin de formule

$R^3OM$

dans laquelle M représente un métal alcalin et $R^3$ a la définition donnée dans la revendication 5, en présence d'un alcool de formule

$R^3OH$

dans laquelle $R^3$ est tel que défini ci-dessus, ou à faire réagir le composé de formule (V) avec un alcoolate de métal alcalin de formule

$R^3OM$

dans laquelle $R^3$ et M sont tels que définis ci-dessus, pour former un composé époxy de formule

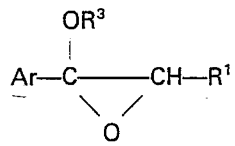

(VII)

dans laquelle Ar, $R^1$ et $R^3$ sont tels que définis ci-dessus, puis à faire réagir le composé époxy avec un alcool de formule

$R^4OH$

dans laquelle $R^4$ est tel que défini dans la revendication 5, en présence d'un alcoolate de métal alcalin de formule

$R^4OM$

dans laquelle $R^4$ est tel que défini ci-dessus et M représente un métal alcalin, ou à faire réagir le composé de formule (V) avec un alkylène-glycol, et

(b) à faire réagir le composé résultant de formule

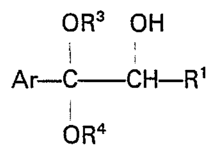

(VI)

dans laquelle Ar, $R^1$, $R^3$ et $R^4$ sont tels que définis ci-dessus, avec un composé de formule

$R^5—SO_2—Hal$ ou $(R^5—SO_2)_2O$

dans laquelle $R^5$ est tel que défini dans la revendication 5 et Hal représente un atome d'halogène.

9. Composé suivant la revendication 6, qui est le diméthylacétal de la 1 - (6 - méthoxy - 2 - naphtyl) - 2 - méthanesulfonyloxy - 1 - propanone.

10. Composé suivant la revendication 6, qui est le diméthylacétal de la 1 - (6 - méthoxy - 2 - naphtyl) - 2 - (d - 10 - camphosulfonyloxy) - 1 - propanone.

11. Composé suivant la revendication 6, qui est le diméthylacétal de la 1 - (6 - méthoxy - 2 - naphtyl) - 2 - (d - 10 - camphosulfonyloxy) - 1 - propanone ayant une rotation optique $[\alpha]_D^{25}$ de +32,5 degrés (C=1, chloroforme).

12. Composé suivant la revendication 6, qui est le diméthylacétal de la 1 - (6 - méthoxy - 2 - naphtyl) - 2 - (d - 10 - camphosulfonyloxy) - 1 - propanone ayant une rotation optique $[\alpha]_D^{25}$ de +4,2 degrés (C=0,143, chloroforme).